# EUROPÄISCHE PATENTSCHRIFT

(11) **EP 4 367 105 B1**
(45) Veröffentlichungstag und Bekanntmachung des Hinweises auf die Patenterteilung: **02.09.2026**
(21) Anmeldenummer: 22743804.1
(22) Anmeldetag: 04.07.2022
(51) Int. Cl.: C07D 271/113, A01N 43/82, A01P 13/00, A01P 21/00

(54) **N-(1,3,4-OXADIAZOL-2-YL)PHENYLCARBOXAMIDE ALS HERBIZIDE**
N-(1,3,4-OXADIAZOL-2-YL)PHENYLCARBOXAMIDES AS HERBICIDES
N-(1,3,4-OXADIAZOL-2-YL)PHENYLCARBOXAMIDES EN TANT QU'HERBICIDES

(30) Priorität: 08.07.2021 EP 21184513
(43) Veröffentlichungstag der Anmeldung: 15.05.2024
(73) Patentinhaber: Bayer Aktiengesellschaft, 51373 Leverkusen (DE)
(72) Erfinder: AHRENS, Hartmut, 51373 Leverkusen (DE); KÖHN, Arnim, 51373 Leverkusen (DE); JAKOBI, Harald, 51373 Leverkusen (DE); WALDRAFF, Christian, 51373 Leverkusen (DE); ASMUS, Elisabeth, 51373 Leverkusen (DE); BOLLENBACH-WAHL, Birgit, 51373 Leverkusen (DE)
(74) Vertreter: BIP Patents
(86) Internationale Anmeldenummer: PCT/EP2022/068441
(87) Internationale Veröffentlichungsnummer: WO 2023/280772

(56) Entgegenhaltungen:
- WO-A1-2012/126932
- WO-A1-2021/094505
- CN-A- 112 694 452

## Beschreibung

Die Erfindung betrifft das technische Gebiet der Herbizide, insbesondere das der Herbizide zur selektiven Bekämpfung von Unkräutern und Ungräsern in Nutzpflanzen.

WO 2012/126932 A1, WO 2017/144402 A1, WO 2018/177871 A1 und CN 112694452 A unter anderem herbizid wirksame Benzoesäureamide, welche am Stickstoffatom der Amidgruppe ein gegebenenfalls substituiertes 1,3,4-Oxadiazol tragen. WO 2021094505 A1 beschreibt herbizid wirksame Benzoesäureamide, die in 4-Position des Phenylrings eine Haloalkoxygruppe tragen und am Stickstoffatom der Amidgruppe unter anderem ein substituiertes 1,3,4-Oxadiazol tragen.

Jedoch weisen die aus diesen Schriften bekannten Benzoesäureamide nicht immer eine ausreichende herbizide Wirkung und/oder Verträglichkeit gegenüber Kulturpflanzen auf. Es wurde gefunden, dass Benzoesäureamide, die
- am Stickstoffatom der Amidgruppe ein unsubstituiertes 1,3,4-Oxadiazol tragen,
- in 4-Position des Phenylrings eine Haloalkoxygruppe tragen
- sowie in 5- und 6-Position des Phenylrings unsubstituiert sind,
überlegene Eigenschaften gegenüber den aus dem Stand der Technik bekannten Benzoesäureamiden aufweisen.

Der Gegenstand der Erfindung wird von den Ansprüchen definiert. Ein

Gegenstand der vorliegenden Erfindung sind somit Benzoesäureamide der Formel (I) oder deren Salze 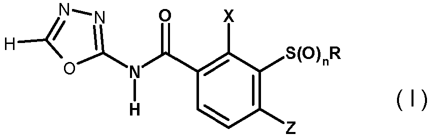 worin die Symbole und Indizes folgende Bedeutungen haben:
- X: bedeutet Cl, Br, Me, Et, MeO, EtO, MeOCH₂ oder MeS,
- Z: bedeutet HF₂CO oder F₃CO,
- R: bedeutet Me oder Et,
- n: bedeutet 0, 1 oder 2.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Durch Halogen substitiertes Alkyl bedeutet geradkettige oder verzweigte Alkylgruppen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Halogen steht für Fluor, Chlor, Brom oder Iod.

Ein heterocyclischer Rest (Heterocyclyl) ist ein 5- oder 6-gliedriger cyclischer Rest, der neben C-Atomen mindestens ein Heteroatom aus der Gruppe N, O, S enthält und der gesättigt, ungesättigt, teilgesättigt oder heteroaromatisch ist und dabei unsubstituiert oder substituiert sein kann, wobei die Bindungsstelle an einem Ringatom lokalisiert ist. Beispiele für heterocyclische Reste sind 1- oder 2- oder 3-Pyrrolidinyl, 3,4-Dihydro-2H-pyrrol-2- oder 3-yl, 2,3-Dihydro-1H-pyrrol-1- oder 2- oder 3- oder 4- oder 5-yl; 2,5-Dihydro-1H-pyrrol-1- oder 2- oder 3-yl, 1- oder 2- oder 3- oder 4-Piperidinyl; 2,3,4,5-Tetrahydropyridin-2- oder 3- oder 4- oder 5-yl oder 6-yl; 1,2,3,6-Tetra-hydropyridin-1- oder 2- oder 3- oder 4- oder 5- oder 6-yl; 1,2,3,4-Tetrahydropyridin-1- oder 2- oder 3- oder 4- oder 5- oder 6-yl; 1,4-Dihydropyridin-1- oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2- oder 3- oder 4-Azepanyl, 2- oder 3-Oxolanyl (= 2- oder 3-Tetrahydrofuranyl); 2,3-Dihydrofuran-2- oder 3- oder 4- oder 5-yl; 2,5-Dihydrofuran-2- oder 3-yl, 2- oder 3- oder 4-Oxanyl (= 2- oder 3- oder 4-Tetrahydropyranyl); 3,4-Dihydro-2H-pyran-2- oder 3- oder 4- oder 5- oder 6-yl; 3,6-Dihydro-2H-pyran-2- oder 3-oder 4- oder 5- oder 6-yl; 2H-Pyran-2- oder 3- oder 4- oder 5- oder 6-yl; 4H-Pyran-2- oder 3- oder 4-yl, 2- oder 3- oder 4-Oxepanyl; 2- oder 3-Tetrahydrothiophenyl; 2,3-Dihydrothiophen-2- oder 3- oder 4- oder 5-yl; 2,5-Dihydrothiophen-2- oder 3-yl; Tetrahydro-2H-thiopyran-2- oder 3- oder 4-yl; 3,4-Dihydro-2H-thiopyran-2- oder 3- oder 4- oder 5- oder 6-yl; 3,6-Dihydro-2H-thiopyran-2- oder 3- oder 4- oder 5- oder 6-yl; 2H-Thiopyran-2- oder 3- oder 4- oder 5- oder 6-yl; 4H-Thiopyran-2- oder 3- oder 4-yl; 1- oder 2- oder 3- oder 4-Pyrazolidinyl; 4,5-Dihydro-3H-pyrazol- 3- oder 4- oder 5-yl; 4,5-Dihydro-1H-pyrazol-1- oder 3- oder 4- oder 5-yl; 2,3-Dihydro-1H-pyrazol-1- oder 2- oder 3- oder 4- oder 5-yl; 1- oder 2- oder 3- oder 4- Imidazolidinyl; 2,3-Dihydro-1H-imidazol-1- oder 2- oder 3- oder 4-yl; 2,5-Dihydro-1H-imidazol-1- oder 2- oder 4- oder 5-yl; 4,5-Dihydro-1H-imidazol-1- oder 2- oder 4- oder 5-yl; Hexahydropyridazin-1- oder 2- oder 3- oder 4-yl; 1,2,3,4-Tetrahydropyridazin-1- oder 2- oder 3- oder 4- oder 5- oder 6-yl; 1,2,3,6-Tetrahydropyridazin-1- oder 2- oder 3- oder 4- oder 5- oder 6-yl; 1,4,5,6-Tetrahydropyridazin-1- oder 3- oder 4- oder 5- oder 6-yl; 3,4,5,6-Tetrahydropyridazin-3- oder 4- oder 5-yl; 4,5-Dihydropyridazin-3- oder 4-yl; 3,4-Dihydropyridazin-3- oder 4- oder 5- oder 6-yl; 3,6-Dihydropyridazin-3- oder 4-yl; 1,6-Dihydropyriazin-1- oder 3- oder 4- oder 5- oder 6-yl; Hexahydropyrimidin-1- oder 2- oder 3- oder 4-yl; 1,4,5,6-Tetrahydropyrimidin-1- oder 2- oder 4- oder 5- oder 6-yl; 1,2,5,6-Tetrahydropyrimidin-1- oder 2- oder 4- oder 5- oder 6-yl; 1,2,3,4-Tetrahydropyrimidin-1- oder 2- oder 3- oder 4- oder 5- oder 6-yl; 1,6-Dihydropyrimidin-1- oder 2- oder 4- oder 5- oder 6-yl; 1,2-Dihydropyrimidin-1- oder 2- oder 4- oder 5- oder 6-yl; 2,5-Dihydropyrimidin-2- oder 4- oder 5-yl; 4,5-Dihydropyrimidin- 4- oder 5- oder 6-yl; 1,4-Dihydropyrimidin-1- oder 2- oder 4- oder 5- oder 6-yl; 1- oder 2- oder 3-Piperazinyl; 1,2,3,6-Tetrahydropyrazin-1- oder 2- oder 3- oder 5- oder 6-yl; 1,2,3,4-Tetrahydropyrazin-1- oder 2- oder 3- oder 4- oder 5- oder 6-yl; 1,2-Dihydropyrazin-1- oder 2- oder 3- oder 5- oder 6-yl; 1,4-Dihydropyrazin-1- oder 2- oder 3-yl; 2,3-Dihydropyrazin-2- oder 3- oder 5- oder 6-yl; 2,5-Dihydropyrazin-2- oder 3-yl; 1,3-Dioxolan-2- oder 4- oder 5-yl; 1,3-Dioxol-2- oder 4-yl; 1,3-Dioxan-2- oder 4- oder 5-yl; 4H-1,3-Dioxin-2- oder 4- oder 5- oder 6-yl; 1,4-Dioxan-2- oder 3- oder 5- oder 6-yl; 2,3-Dihydro-1,4-dioxin-2- oder 3- oder 5- oder 6-yl; 1,4-Dioxin-2- oder 3-yl; 1,2-Dithiolan-3- oder 4-yl; 3H-1,2-Dithiol-3- oder 4- oder 5-yl; 1,3-Dithiolan-2- oder 4-yl; 1,3-Dithiol-2- oder 4-yl; 1,2-Dithian-3- oder 4-yl; 3,4-Dihydro-1,2-dithiin-3- oder 4- oder 5- oder 6-yl; 3,6-Dihydro-1,2-dithiin-3- oder 4-yl; 1,2-Dithiin-3- oder 4-yl; 1,3-Dithian-2- oder 4- oder 5-yl; 4H-1,3-Dithiin-2- oder 4- oder 5- oder 6-yl; Isoxazolidin-2- oder 3- oder 4- oder 5-yl; 2,3-Dihydroisoxazol-2- oder 3- oder 4- oder 5-yl; 2,5-Dihydroisoxazol-2- oder 3- oder 4- oder 5-yl; 4,5-Dihydroisoxazol-3- oder 4- oder 5-yl; 1,3-Oxazolidin-2- oder 3- oder 4- oder 5-yl; 2,3-Dihydro-1,3-oxazol-2- oder 3- oder 4- oder 5-yl; 2,5-Dihydro-1,3-oxazol-2- oder 4- oder 5-yl; 4,5-Dihydro-1,3-oxazol-2- oder 4- oder 5-yl; 1,2-Oxazinan-2- oder 3- oder 4- oder 5- oder 6-yl; 3,4-Dihydro-2H-1,2-oxazin-2- oder 3- oder 4- oder 5- oder 6-yl; 3,6-Dihydro-2H-1,2-oxazin-2- oder 3- oder 4- oder 5- oder 6-yl; 5,6-Dihydro-2H-1,2-oxazin-2- oder 3- oder 4- oder 5- oder 6-yl; 5,6-Dihydro-4H-1,2-oxazin-3- oder 4- oder 5- oder 6-yl; 2H-1,2-Oxazin-2- oder 3- oder 4- oder 5- oder 6-yl; 6H-1,2-Oxazin-3- oder 4- oder 5- oder 6-yl; 4H-1,2-Oxazin-3- oder 4- oder 5- oder 6-yl; 1,3-Oxazinan-2- oder 3- oder 4- oder 5- oder 6-yl; 3,4-Dihydro-2H-1,3-oxazin-2- oder 3- oder 4- oder 5- oder 6-yl; 3,6-Dihydro-2H-1,3-oxazin-2- oder 3- oder 4- oder 5- oder 6-yl; 5,6-Dihydro-2H-1,3-oxazin-2- oder 4- oder 5- oder 6-yl; 5,6-Dihydro-4H-1,3-oxazin-2- oder 4- oder 5- oder 6-yl; 2H-1,3-Oxazin-2- oder 4- oder 5- oder 6-yl; 6H-1,3-Oxazin-2- oder 4- oder 5- oder 6-yl; 4H-1,3-Oxazin-2- oder 4- oder 5- oder 6-yl; Morpholin-2- oder 3- oder 4-yl; 3,4-Dihydro-2H-1,4-oxazin-2- oder 3- oder 4- oder 5- oder 6-yl; 3,6-Dihydro-2H-1,4-oxazin-2- oder 3- oder 5- oder 6-yl; 2H-1,4-oxazin-2- oder 3- oder 5- oder 6-yl; 4H-1,4-oxazin-2- oder 3-yl; Isothiazolidin-2- oder 3- oder 4- oder 5-yl; 2,3-Dihydroisothiazol-2- oder 3- oder 4- oder 5-yl; 2,5-Dihydroisothiazol-2- oder 3- oder 4- oder 5-yl; 4,5-Dihydroisothiazol-3- oder 4- oder 5-yl; 1,3-Thiazolidin-2- oder 3- oder 4- oder 5-yl; 2,3-Dihydro-1,3-thiazol-2- oder 3- oder 4- oder 5-yl; 2,5-Dihydro-1,3-thiazol-2- oder 4- oder 5-yl; 4,5-Dihydro-1,3-thiazol-2- oder 4- oder 5-yl; 1,3-Thiazinan-2- oder 3- oder 4- oder 5- oder 6-yl; 3,4-Dihydro-2H-1,3-thiazin-2- oder 3- oder 4- oder 5- oder 6-yl; 3,6-Dihydro-2H-1,3-thiazin-2- oder 3- oder 4- oder 5- oder 6-yl; 5,6-Dihydro-2H-1,3-thiazin-2- oder 4- oder 5- oder 6-yl; 5,6-Dihydro-4H-1,3-thiazin-2- oder 4- oder 5- oder 6-yl; 2H-1,3-Thiazin-2- oder 4- oder 5- oder 6-yl; 6H-1,3-Thiazin-2- oder 4- oder 5- oder 6-yl; 4H-1,3-Thiazin-2- oder 4- oder 5- oder 6-yl; 4,2-Dioxazolidin-2- oder 3- oder 5-yl; 1,4,2-Dioxazol-3- oder 5-yl; 1,4,2-Dioxazinan-2- oder -3- oder 5- oder 6-yl; 5,6-Dihydro-1,4,2-dioxazin-3- oder 5- oder 6-yl; 1,4,2-Dioxazin-3- oder 5- oder 6-yl.

Die Verbindungen der allgemeinen Formel (I) können je nach Art und Verknüpfung der Substituenten als Stereoisomere vorliegen. Sind beispielsweise ein oder mehrere asymmetrisch substituierte Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Die Verbindungen der Formel (I) können Salze bilden. Geeignete Basen sind beispielsweise organische Amine, wie Trialkylamine, Morpholin, Piperidin oder Pyridin sowie Ammonium-, Alkali- oder Erdalkalimetallhydroxide, -carbonate und -hydrogen-carbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R‴]⁺, worin R bis R‴ jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Erfindungsgemäße Verbindungen können beispielsweise nach den in WO 2012/126932 A1, WO 2017/144402 A1, WO 2018/177871 A1 und WO 2021094505 A1 angegebenen Methoden hergestellt werden. Die entsprechenden Benzoesäurechloride, Benzoesäureester beziehungsweise die ihnen zugrunde liegenden Benzoesäuren sind grundsätzlich bekannt und können beispielsweise gemäß der in WO 2021094505 A1 beschriebenen Methoden hergestellt werden. Die weiter unten beschriebenen Ausführungsbeispiele erläutern die Herstellungsweise der erfindungsgemäßen Verbindungen näher.

Die Aufarbeitung der jeweiligen Reaktionsmischungen erfolgt in der Regel nach bekannten Verfahren, beispielsweise durch Kristallisation, wässrig-extraktive Aufarbeitung, durch chromatographische Methoden oder durch Kombination dieser Methoden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Die erfindungsgemäßen Verbindungen können in Nutzkulturen Selektivitäten aufweisen und können auch als nichtselektive Herbizide eingesetzt werden.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten in der Agrarindustrie verwendeten Wirkstoff , vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften liegen in einer Toleranz oder Resistenz gegen abiotische Stressoren z.B. Hitze, Kälte, Trockenheit, Salz und ultraviolette Strahlung.

Bevorzugt ist die Anwendung der erfindungsgemäßen Verbindungen der Formel (I) oder deren Salze in wirtschaftlich bedeutenden transgenen Kulturen von Nutz-und Zierpflanzen,

Die Verbindungen der Formel (I) können als Herbizide in Nutzpflanzenkulturen eingesetzt werden, welche gegenüber den phytotoxischen Wirkungen der Herbizide resistent sind bzw. gentechnisch resistent gemacht wurden.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044,

EP 0131624). Beschrieben wurden beispielsweise in mehreren Fällen gentechnische Veränderungen von Kulturpflanzen zwecks Modifikation der in den Pflanzen synthetisierten Stärke (z.B. WO 92/011376 A, WO 92/014827 A, WO 91/019806 A), transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung OptimumTM GATTM (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen. So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4-D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung OptimumTM GATTM (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxid-addukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen Wirkstoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die Verbindungen der allgemeinen Formel (I) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen oder als Pflanzenwuchsregulatoren wirken, einsetzbar, wie sie z.B. aus Weed Research 26 (1986) 441-445 oder "The Pesticide Manual", 14th edition, The British Crop Protection Council and the Royal Soc. of Chemistry, 2006 und dort zitierter Literatur beschrieben sind.

Als bekannte Herbizide oder Pflanzenwachstumsregulatoren, die mit Verbindungen der allgemeinen Formel (I) kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acifluorfen, Acifluorfen-methyl, Acifluorfen-Natrium, Aclonifen, Alachlor, Allidochlor, Alloxydim, Alloxydim-Natrium, Ametryn, Amicarbazon, Amidochlor, Amidosulfuron, 4-Amino-3-chlor-6-(4-chlor-2-fluor-3-methylphenyl)-5-fluorpyridin-2-carbonsäure, Aminocyclopyrachlor, Aminocyclopyrachlor-Kalium, Aminocyclopyrachlor-methyl, Aminopyralid, Aminopyraliddimethylammonium, Aminopyralid-tripromine, Amitrol, Ammoniumsulfamate, Anilofos, Asulam, Asulam-Kalium, Asulam-Natrium, Atrazin, Azafenidin, Azimsulfuron, Beflubutamid, (S)-(-)-Beflubutamid, Beflubutamid-M, Benazolin, Benazolin-ethyl, Benazolin-dimethylammonium, Benazolin-Klaium, Benfluralin, Benfuresate, Bensulfuron, Bensulfuron-methyl, Bensulid, Bentazon, Bentazon-Natrium, Benzobicyclon, Benzofenap, Bicyclopyrone, Bifenox, Bilanafos, Bilanafos-Natium, Bipyrazone, Bispyribac, Bispyribac-Natium, Bixlozon, Bromacil, Bromacil-lithium, Bromacil-Natrium, Bromobutid, Bromofenoxim, Bromoxynil, Bromoxynilbutyrat, Bromoxynil-Kalium, Bromoxynilheptanoat und Bromoxynil-octanoat, Busoxinon, Butachlor, Butafenacil, Butamifos, Butenachlor, Butralin, Butroxydim, Butylat, Cafenstrol, Cambendichlor, Carbetamide, Carfentrazon, Carfentrazon-Ethyl, Chloramben, Chloramben-ammonium, Chloramben-diolamin, Chlroamben-methyl, Chlorambenmethylammonium, Chloramben-Natium, Chlorbromuron, Chlorfenac, Chlorfenac-ammonium, Chlorfenac-Natium, Chlorfenprop, Chlorfenprop-methyl, Chlorflurenol, Chlorflurenol-methyl, Chloridazon, Chlorimuron, Chlorimuron-ethyl, Chlorophthalim, Chlorotoluron, Chlorsulfuron, Chlorthal, Chlorthal-dimethyl, Chlorthal-monomethyl, Cinidon, Cinidon-ethyl, Cinmethylin, exo-(+)-Cinmethylin, d.h. (1R,2S,4S)-4-isopropyl-1-methyl-2-[(2-methylbenzyl)oxy]-7-oxabicyclo[2.2.1]heptan, exo-(-)-Cinmethylin, d.h. (1R,2S,4S)-4-isopropyl-1-methyl-2-[(2-methylbenzyl)oxy]-7-oxabicyclo[2.2.1]heptan, Cinosulfuron, Clacyfos, Clethodim, Clodinafop, Clodinafop-ethyl, Clodinafoppropargyl, Clomazon, Clomeprop, Clopyralid, Clopyralid-methyl, Clopyralid-olamin, Clopyralid-Kalium, Clopyralid-tripomin, Cloransulam, Cloransulam-methyl, Cumyluron, Cyanamide, Cyanazine, Cycloat, Cyclopyranil, Cyclopyrimorat, Cyclosulfamuron, Cycloxydim, Cyhalofop, Cyhalofop-butyl, Cyprazin, 2,4-D (sowie die Ammonium, Butotyl, Butyl, Cholin, Diethylammonium, Dimethylammonium, Diolamin, Doboxyl, Dodecylammonium, Etexyl, Ethyl, 2-Ethylhexyl, Heptylammonium, Isobutyl, Isooctyl, Isopropyl, Isopropylammonium, Lithium, Meptyl, Methyl, Kalium, Tetradecylammonium, Triethylammonium, Triisopropanolammonium, Tripromin and Trolamin Salze davon), 2,4-DB, 2,4-DB-butyl, 2,4-DB-Dimethylammonium, 2,4-DB-isooctyl, 2,4-DB-Kalium und 2,4-DB-Natrium, Daimuron (Dymron), Dalapon, Dalapon-Calcium, Dalapon-Magnesium, Dalapon-Natium, Dazomet, Dazomet-Natrium, n-Decanol, 7-Deoxy-D-sedoheptulose, Desmedipham, Detosylpyrazolat (DTP), Dicamba und seine Salze (z.B. Dicamba-biproamin, Dicamba-N,N-Bis(3-aminopropyl)methylamin, Dicamba-butotyl, Dicamba-cholin, Dicamba-Diglycolamin, Dicamba-Dimethylammonium, Dicamba-Diethanolaminemmonium, Dicamba-Diethylammonium, Dicambaisopropylammonium, Dicamba-methyl, Dicamba-monoethanolamin, Dicamba-olamin, Dicamba-Kalium, Dicamba-Natium, Dicamba-Triethanolamin), Dichlobenil, 2-(2,4-Dichlorbenzyl)-4,4-dimethyl-1,2-oxazolidin-3-on, 2-(2,5-Dichlorbenzyl)-4,4-dimethyl-1,2-oxazolidin-3-one, Dichlorprop, Dichlorprop-butotyl, Dichlorprop-Dimethylammonium, Dichhlorprop-etexyl, Dichlorpropethylammonium, Dichlorprop-isoctyl, Dichlorprop-methyl, Dichlorprop-Kalium, Dichlorprop-Natrium, Dichlorprop-P, Dichlorprop-P-Dimethylammonium, Dichlorprop-P-etexyl, Dichlorprop-P-Kalium, Dichlorprop-Natrium, Diclofop, Diclofop-methyl, Diclofop-P, Diclofop-P-methyl, Diclosulam, Difenzoquat, Difenzoquat-metilsulfate, Diflufenican, Diflufenzopyr, Diflufenzopyr- Natrium, Dimefuron, Dimepiperate, Dimesulfazet, Dimethachlor, Dimethametryn, Dimethenamid, Dimethenamid-P, Dimetrasulfuron, Dinitramine, Dinoterb, Dinoterb-Acetate, Diphenamid, Diquat, Diquat-Dibromid, Diquat-Dichloride, Dithiopyr, Diuron, DNOC, DNOC-Ammonium, DNOC-Kalium, DNOC-Natrium, Endothal, Endothal-Diammonium, Endothal-Dikalium, Endothal-Dinatrium, Epyrifenacil (S-3100), EPTC, Esprocarb, Ethalfluralin, Ethametsulfuron, Ethametsulfuron-Methyl, Ethiozin, Ethofumesate, Ethoxyfen, Ethoxyfen-Ethyl, Ethoxysulfuron, Etobenzanid, F-5231, d.h. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, Fenoxaprop, Fenoxaprop-P, Fenoxaprop-Ethyl, Fenoxaprop-P-Ethyl, Fenoxasulfone, Fenpyrazone, Fenquinotrione, Fentrazamid, Flamprop, Flamprop-Isoproyl, Flamprop-Methyl, Flamprop-M-Isopropyl, Flamprop-M-Methyl, Flazasulfuron, Florasulam, Florpyrauxifen, Florpyrauxifen-benzyl, Fluazifop, Fluazifop-Butyl, Fluazifop-Methyl, Fluazifop-P, Fluazifop-P-Butyl, Flucarbazone, Flucarbazone-Natrium, Flucetosulfuron, Fluchloralin, Flufenacet, Flufenpyr, Flufenpyr-Ethyl, Flumetsulam, Flumiclorac, Flumiclorac-Pentyl, Flumioxazin, Fluometuron, Flurenol, Flurenol-Butyl, -Dimethylammonium und -Methyl, Fluoroglycofen, Fluoroglycofen-Ethyl, Flupropanat, Flupropanat-Natrium, Flupyrsulfuron, Flupyrsulfuron-Methyl, Flupyrsulfuron-Methyl-Natrium, Fluridon, Flurochloridon, Fluroxypyr, Fluroxypyr-Butometyl, Fluroxypyr-Meptyl, Flurtamon, Fluthiacet, Fluthiacet-Methyl, Fomesafen, Fomesafen-Natrium, Foramsulfuron, Foramsulfuron-Natrium, Fosamine, Fosamine-Ammonium, Glufosinat, Glufosinat-Ammonium, Glufosinat-Natrium, L-Glufosinat-Ammonium, L-Glufosinat-Natrium, Glufosinat-P-Natrium, Glufosinat-P-Ammonium, Glyphosat, Glyphosat-Ammonium, Glyphosat-Isopropylammonium, Glyphosat-Diammonium, Glyphosat-Dimethylammonium, Glyphosat-Kalium, Glyphosat-Natrium, Glyphosat-Sesquinatrium und Glyphosat-Trimesium, H-9201, d.h. O-(2,4-Dimethyl-6-nitrophenyl)-O-ethyl-isopropylphosphoramidothioat, Halauxifen, Halauxifen-methyl, Halosafen, Halosulfuron, Halosulfuron-Methyl, Haloxyfop, Haloxyfop-P, Haloxyfop-Ethoxyethyl, Haloxyfop-P-Ethoxyethyl, Haloxyfop-Methyl, Haloxyfop-P-Methyl, Haloxifop-Natrium, Hexazinon, HNPC-A8169, i.e. Prop-2-yn-1-yl (2S)-2-{3-[(5-tert-butylpyridin-2-yl)oxy]phenoxy}propanoat, HW-02, d.h. 1-(Dimethoxyphosphoryl)-ethyl-(2,4-dichlorphenoxy)acetat, Hydantocidin, Imazamethabenz, Imazamethabenz-Methyl, Imazamox, Imazamox-Ammonium, Imazapic, Imazapic-Ammonium, Imazapyr, Imazapyr-Isopropylammonium, Imazaquin, Imazaquin-Ammonium, Imazaquin-Methyl, Imazethapyr, Imazethapyr-Ammonium, Imazosulfuron, Indanofan, Indaziflam, Iodosulfuron, Iodosulfuron-Methyl, Iodosulfuron-Methyl-Natrium, Ioxynil, Ioxynil-Lithium, -Octanoat, -Kalium und Natrium, Ipfencarbazon, Isoproturon, Isouron, Isoxaben, Isoxaflutole, Karbutilat, KUH-043, d.h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Ketospiradox, Ketospiradox-Kalium, Lactofen, Lenacil, Linuron, MCPA, MCPA-Butotyl, -Butyl, -Dimethylammonium, -Diolamin, -2-Ethylhexyl, -Ethyl, -Isobutyl, Isoctyl, -Isopropyl, -Isopropylammonium, - Methyl, Olamin, -Kalium, -Natrium und -Trolamin, MCPB, MCPB-Methyl, -Ethyl und -Natrium, Mecoprop, Mecoprop-Butotyl, Mecoprop- dimethylammonium, Mecoprop-Diolamin, Mecoprop-Etexyl, Mecoprop-Ethadyl, Mecoprop-Isoctyl, Mecoprop-Methyl, Mecoprop-Kalium, Mecoprop-Natrium, und Mecoprop-Trolamin, Mecoprop-P, Mecoprop-P-Butotyl, -Dimethylammonium, -2-Ethylhexyl und - Kalium, Mefenacet, Mefluidid, Mefluidid-Diolamin, Mefluidid-Kalium, Mesosulfuron, Mesosulfuron-Methyl, Mesosulfuron-Natrium, Mesotrion, Methabenzthiazuron, Metam, Metamifop, Metamitron, Metazachlor, Metazosulfuron, Methabenzthiazuron, Methiopyrsulfuron, Methiozolin, Methyl isothiocyanat, Metobromuron, Metolachlor, S-Metolachlor, Metosulam, Metoxuron, Metribuzin, Metsulfuron, Metsulfuron-Methyl, Molinat, Monolinuron, Monosulfuron, Monosulfuron-Methyl, MT-5950, d.h. N-[3-Chlor-4-(1-methylethyl)-phenyl]-2-methylpentanamid, NGGC-011, Napropamid, NC-310, i.e. 4-(2,4-Dichlorbenzoyl)-1-methyl-5-benzyloxypyrazol, NC-656, i.e. 3-[(Isopropylsulfonyl)methyl]-N-(5-methyl-1,3,4-oxadiazol-2-yl)-5-(trifluormethyl)[1,2,4]triazolo-[4,3-a]pyridin-8-carboxamid, Neburon, Nicosulfuron, Nonansäure (Pelargonsäure), Norflurazon, Ölsäure (Fettsäuren), Orbencarb, Orthosulfamuron, Oryzalin, Oxadiargyl, Oxadiazon, Oxasulfuron, Oxaziclomefone, Oxyfluorfen, Paraquat, Paraquat-dichlorid, Paraquat-Dimethylsulfat, Pebulat, Pendimethalin, Penoxsulam, Pentachlorphenol, Pentoxazon, Pethoxamid, Petroleumöl, Phenmedipham, Phenmedipham-Ethyl, Picloram, Picloram-dimethylammonium, Picloram-Etexyl, Picloram-Isoctyl, Picloram-Methyl, Picloram-Olamin, Picloram-Kalium, Picloram-Triethylammonium, Picloram-Tripromin, Picloram-Trolamin, Picolinafen, Pinoxaden, Piperophos, Pretilachlor, Primisulfuron, Primisulfuron-Methyl, Prodiamine, Profoxydim, Prometon, Prometryn, Propachlor, Propanil, Propaquizafop, Propazine, Propham, Propisochlor, Propoxycarbazone, Propoxycarbazone-Natrium, Propyrisulfuron, Propyzamid, Prosulfocarb, Prosulfuron, Pyraclonil, Pyraflufen, Pyraflufen-Ethyl, Pyrasulfotol, Pyrazolynat (Pyrazolat), Pyrazosulfuron, Pyrazosulfuron-Ethyl, Pyrazoxyfen, Pyribambenz, Pyribambenz-Isopropyl, Pyribambenz-Propyl, Pyribenzoxim, Pyributicarb, Pyridafol, Pyridat, Pyriftalid, Pyriminobac, Pyriminobac-Methyl, Pyrimisulfan, Pyrithiobac, Pyrithiobac-Natrium, Pyroxasulfon, Pyroxsulam, Quinclorac, Quinclorac-Dimethylammonium, Quinclorac-Methyl, Quinmerac, Quinoclamin, Quizalofop, Quizalofop-Ethyl, Quizalofop-P, Quizalofop-P-Ethyl, Quizalofop-P-Tefuryl, QYM201, i.e. 1-{2-Chlor-3-[(3-cyclopropyl-5-hydroxy-1-methyl-1H-pyrazol-4-yl)carbonyl]-6-(trifluormethyl)phe-nyl}piperidin-2-on, Rimsulfuron, Saflufenacil, Sethoxydim, Siduron, Simazine, Simetryn, SL-261, Sulcotrione, Sulfentrazone, Sulfometuron, Sulfometuron-Methyl, Sulfosulfuron, , SYP-249, d.h. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trichloressigsäure) und seine Salze, z.B. TCA-ammonium, TCA-Calcium, TCA-Ethyl, TCA-Magnesium, TCA-Natrium, Tebuthiuron, Tefuryltrione, Tembotrion, Tepraloxydim, Terbacil, Terbucarb, Terbumeton, Terbuthylazine, Terbutryn, Tetflupyrolimet, Thaxtomin, Thenylchlor, Thiazopyr, Thiencarbazone, Thiencarbazon-Methyl, Thifensulfuron, Thifensulfuron-Methyl, Thiobencarb, Tiafenacil, Tolpyralat, Topramezon, Tralkoxydim, Triafamon, Tri-allat, Triasulfuron, Triaziflam, Tribenuron, Tribenuron-Methyl, Triclopyr, Triclopyr-Butotyl, Triclopyr-Cholin, Triclopyr-Ethyl, Triclopyr-Triethylammonium, Trietazine, Trifloxysulfuron, Trifloxysulfuron-Natrium, Trifludimoxazin, Trifluralin, Triflusulfuron, Triflusulfuron-Methyl, Tritosulfuron, Harnstoffsulfat, Vernolat, XDE-848, ZJ-0862, d.h. 3,4-Dichlor-N-{2-[(4,6-dimethoxypyrimidin-2-yl)oxy]benzyl}anilin, 3-(2-Chlor-4-fluor-5-(3-methyl-2,6-dioxo-4-trifluormethyl-3,6-dihydropyrimidin-1(2H)-yl)phenyl)-5-methyl-4,5-dihydroisoxazole-5-carbonsäureethylester, Ethyl-[(3-{2-chlor-4-fluor-5-[3-methyl-2,6-dioxo-4-(trifluormethyl)-3,6-dihydropyrimidin-1(2H)-yl]phenoxy}pyridin-2-yl)oxy]acetat, 3-Chlor-2-[3-(difluormethyl)isoxazolyl-5-yl]phenyl-5-chlorpyrimidin-2-ylether, 2-(3,4-Dimethoxyphenyl)-4-[(2-hydroxy-6-oxocyclohex-1-en-1-yl)carbonyl]-6-methylpyridazine-3(2H)-on, 2-({2-[(2-Methoxyethoxy)methyl]-6-methylpyridin-3-yl}carbonyl)cyclohexane-1,3-dion, (5-Hydroxy-1-methyl-1H-pyrazol-4-yl)(3,3,4-trimethyl-1,1-dioxido-2,3-dihydro-1-benzothiophen-5-yl)methanon, 1-Methyl-4-[(3,3,4-trimethyl-1,1-dioxido-2,3-dihydro-1-benzothiophen-5-yl)carbonyl]-1H-pyrazol-5-yl propan-1-sulfonat, 4-{2-Chlor-3-[(3,5-dimethyl-1H-pyrazol-1-yl)methyl]-4-(methylsulfonyl)benzoyl}-1-methyl-1H-pyrazol-5-yl-1,3-dimethyl-1H-pyrazol-4-carboxylat; Cyanomethyl-4-amino-3-chlor-5-fluor-6-(7-fluor-1H-indol-6-yl)pyridin-2-carboxylat, Prop-2-yn-1-yl 4-amino-3-chlor-5-fluor-6-(7-fluor-1H-indol-6-yl)pyridin-2-carboxylat, Methyl-4-amino-3-chlor-5-fluor-6-(7-fluor-1H-indol-6-yl)pyridin-2-carboxylat, 4-Amino-3-chlor-5-fluor-6-(7-fluor-1H-indol-6-yl)pyridin-2-carbonsäure, Benzyl-4-amino-3-chlor-5-fluor-6-(7-fluor-1H-indol-6-yl)pyridin-2-carboxylat, Ethyl-4-amino-3-chlor-5-fluor-6-(7-fluor-1H-indol-6-yl)pyridin-2-carboxylat, Methyl-4-amino-3-chlor-5-fluor-6-(7-fluor-1-isobutyryl-1H-indol-6-yl)pyridin-2-carboxylat, Methyl 6-(1-acetyl-7-fluor-1H-indol-6-yl)-4-amino-3-chlor-5-fluorpyridin-2-carboxylat, Methyl-4-amino-3-chlor-6-[1-(2,2-dimethylpropanoyl)-7-fluor-1H-indol-6-yl]-5-fluorpyridin-2-carboxylat, Methyl-4-amino-3-chlor-5-fluor-6-[7-fluor-1-(methoxyacetyl)-1H-indol-6-yl]pyridin-2-carboxylat, Kalium 4-amino-3-chlor-5-fluor-6-(7-fluor-1H-indol-6-yl)pyridin-2-carboxylat, Natrium-4-amino-3-chlor-5-fluor-6-(7-fluor-1H-indol-6-yl)pyridin-2-carboxylat, Butyl-4-amino-3-chlor-5-fluoro-6-(7-fluoro-1H-indol-6-yl)pyridin-2-carboxylat, 4-Hydroxy-1-methyl-3-[4-(trifluoromethyl)pyridin-2-yl]imidazolidin-2-on, 3-(5-tert-butyl-1,2-oxazol-3-yl)-4-hydroxy-1-methylimidazolidin-2-on, 3-[5-Chlor-4-(trifluormethyl)pyridin-2-yl]-4-hydroxy-1-methylimidazolidin-2-on, 4-Hydroxy-1-methoxy-5-methyl-3-[4-(trifluormethyl)pyridin-2-yl]imidazolidin-2-on, 6-[(2-Hydroxy-6-oxocyclohex-1-en-1-yl)carbonyl]-1,5-dimethyl-3-(2-methylphenyl)chinazolin-2,4(1H,3H)-dion, 3-(2,6-Dimethylphenyl)-6-[(2-hydroxy-6-oxocyclohex-1-en-1-yl)carbonyl]-1-methylchinazolin-2,4(1H,3H)-dion, 2-[2-chlor-4-(methylsulfonyl)-3-(morpholin-4-ylmethyl)benzoyl]-3-hydroxycyclohex-2-en-1-on, 1-(2-carboxyethyl)-4-(pyrimidin-2-yl)pyridazin-1-iumsalz (mit passenden Anionen wie z.B Chlorid, Acetat oder Trifluoracetat), 1-(2-Carboxyethyl)-4-(pyridazin-3-yl)pyridazin-1-iumsalz (mit passenden Anionen wie z.B. Chlorid, Acetat oder Trifluoracetat), 4-(Pyrimidin-2-yl)-1-(2-sulfoethyl)pyridazin-1-ium salz iumsalz (mit passenden Anionen wie z.B Chlorid, Acetat oder Trifluoracetat), 4-(Pyridazin-3-yl)-1-(2-sulfoethyl)pyridazin-1-iumsalz (mit passenden Anionen wie z.B Chlorid, Acetat oder Trifluoracetat), 1-(2-Carboxyethyl)-4-(1,3-thiazol-2-yl)pyridazin-1-iumsalz (mit passenden Anionen wie z.B Chlorid, Acetat oder Trifluoracetat), 1-(2-Carboxyethyl)-4-(1,3-thiazol-2-yl)pyridazin-1-ium salz (mit passenden Anionen wie z.B Chlorid, Acetat oder Trifluoracetat), Methyl (2R)-2-{[(E)-({2-chlor-4-fluor-5-[3-methyl-2,6-dioxo-4-(trifluormethyl)-3,6-dihydropyrimidin-1(2H)-yl]phenyl}methyliden)amino]oxy}propanoat, (E)-2-(Trifluormethyl)benzaldehyde O-{2,6-bis[(4,6-dimethoxypyrimidin-2-yl)oxy]benzoyl}oxim, 2-Fluor-N-(5-methyl-1,3,4-oxadiazol-2-yl)-3-[(R)-propylsulfinyl]-4-(trifluormethyl)benzamid, (2R)-2-[(4-Amino-3,5-dichlor-6-fluor-2-pyridyl)oxy]propancarbonsäure.

Beispiele für Pflanzenwachstumsregulatoren als mögliche Mischungspartner sind:
Abscisinsäure und verwandte Analoga [z.B. (2Z,4E)-5-[6-Ethynyl-1-hydroxy-2,6-dimethyl-4-oxocyclohex-2-en-1-yl]-3-methylpenta-2,4-diensäure, methyl-(2Z,4E)-5-[6-ethynyl-1-hydroxy-2,6-dimethyl-4-oxocyclohex-2-en-1-yl]-3-methylpenta-2,4-dienoat, (2Z,4E)-3-ethyl-5-(1-hydroxy-2,6,6-trimethyl-4-oxocyclohex-2-en-1-yl)penta-2,4-diensäure, (2E,4E)-5-(1-hydroxy-2,6,6-trimethyl-4-oxocyclohex-2-en-1-yl)-3-(trifluoromethyl)penta-2,4-diensäure, methyl (2E,4E)-5-(1-hydroxy-2,6,6-trimethyl-4-oxocyclohex-2-en-1-yl)-3-(trifluoromethyl)penta-2,4-dienoat, (2Z,4E)-5-(2-hydroxy-1,3-dimethyl-5-oxobicyclo[4.1.0]hept-3-en-2-yl)-3-methylpenta-2,4-diensäure], Acibenzolar, Acibenzolar-S-methyl, S-Adenosylhomocystein, Allantoin, 2-Aminoethoxyvinylglycin (AVG), Aminooxyessigsäure and verwandte Ester [z.B. (Isopropyliden)-aminooxyessigsäure-2-(methoxy)-2-oxoethylester, (Isopropyliden)-aminooxyessigsäure-2-(hexyloxy)-2-oxoethylester, (Cyclohexyliden)-aminooxyessigsäure-2-(isopropyloxy)-2-oxoethylester], 1-Aminocycloprop-1-ylcarbonsäure N-Methyl-1-aminocyclopropyl-1-carbonsäure, 1-Aminocyclopropyl-1-carbonsäureamid, substituierte 1-Aminocyclopropyl-1-carbonsäurederivate wie sie in DE3335514, EP30287, DE2906507 oder US5123951 beschrieben werden, 1-Aminocyclopropyl-1-hydroxamsäure, 5-Aminolevulinsäure, Ancymidol, 6-Benzylaminopurin, Bikinin, Brassinolid, Brassinolide-ethyl, L-Canalin, Catechin und catechine (z.B. (2S,3R)-2-(3,4-Dihydroxyphenyl)-3,4-dihydro-2H-chromen-3,5,7-triol), Chitooligosaccharides (CO; COs unterscheiden sich von LCOs dadurch, daß ihnen die für LCOs charakteristische Fettsäureseitenkette fehlt. COs, in manchen Fällen als N-Acetylchitooligosaccharide bezeichnet, sind auch aus GlcNAc-Einheiten aufgebaut, aber haben Seitenketten, durch die sies ich von Chitinmolekülen unterscheiden [(C₈H₁₃NO₅)ₙ, CAS No. 1398-61-4] und chitosan Moleküle [(C₃H₁₁NO₄)ₙ, CAS No. 9012-76-4]), Chitin-artige Verbindungen, Chlormequat chloride, Cloprop, Cyclanilide, 3-(Cycloprop-1-enyl)propionsäure, 1-[2-(4-Cyano-3,5-dicyclopropylphenyl)acetamido]cyclohexancarbonsäure, 1-[2-(4-Cyano-3-cyclopropylphenyl)acetamido]cyclohexancarbonsäure, 1-Cyclopropenylmethanol, Daminozid, Dazomet, Dazomet-Natrium, n-Decanol, Dikegulac, Dikegulac-Natrium, Endothal, Endothal-diKalium, -di-Natrium, und mono(N,N-dimethylalkylammonium), Ethephon, 1-Ethylcyclopropen,Flumetralin, Flurenol, Flurenol-butyl, Flurenol-methyl, Flurprimidol, Forchlorfenuron, Gibberellinsäure, Inabenfid, Indol-3-essigsäure (IAA), 4-Indol-3-ylbuttersäure, Isoprothiolan, Probenazole, Jasmonsäure, Jasmonsäureester oder andere Derivate (z.B. Jasmonsäuremethylester, Jasmonsäureethylester), Lipochitooligosaccharide (LCO, in manchen Fällen auch als Symbiotische Nodulationssignale (Nod oder Nod Faktoren) oder als Myc Faktoren bezeichnet, bestehen aus einem Oligosacchariderückgrat aus β-1,4-verknüpften N-Acetyl-D-Glucosaminresten ("GlcNAc") mit einer N-verknüpften Fettsäureseitenkette, die am nicht reduzierenden Ende ankondensiert ist. Wie aus der Literatur zu entnehmen ist, unterscheiden sich LCOs in der Zahl an GlcNAc-EInheiten in der Rückgratstruktur, in der Länge und dem Sättigungsgrad der Fettsäurekette sowie in der Substitution der reduzierenden und nicht-reduzierenden Zuckereinheiten), Linoleinsäure oder ihre Derivate, Linolensäure oder ihre Derivate, Maleinsäurehydrazid, Mepiquatchlorid, Mepiquatpentaborat, 1-Methylcyclopropen, 3-Methylcyclopropen, Methoxyvinylglycin (MVG), 3'-Methylabscisinsäure, 1-(4-Methylphenyl)-N-(2-oxo-1-propyl-1,2,3,4-tetrahydrochinolin-6-yl)methansulfonamid und verwandte substituierte (Tetrahydrochinolin-6-yl)methansulfonamide, (3E,3aR,8bS)-3-({[(2R)-4-Methy1-5-oxo-2,5-dihydrofuran-2-yl]oxy}methylen)-3,3a,4,8b-tetrahydro-2H-indeno[1,2-b]furan-2-on und verwandte Laktone wie sie in EP2248421 beschrieben sind, 2-(1-Naphthyl)acetamid, 1-Naphthylessigsäure, 2- Naphthyloxyessigsäure, Nitrophenolatmischung, 4-Oxo-4[(2-phenylethyl)amino]buttersäure, Paclobutrazol, 4-Phenylbuttersäure and ihre Salze (z.B. Natrium-4-phenylbutanoat, Kalium-4-phenylbutanoat), Phenylalanine, N-Phenylphthalamsäure, Prohexadione, Prohexadion-Calcium, , 1-n-Propylcyclopropen, Putrescin, Prohydrojasmon, Rhizobitoxin, Salicylsäure und Salicyclsäuremethylester, Sarcosin, Natriumcycloprop-1-en-1-ylacetat, Natriumcycloprop-2-en-1-ylacetat, Natrium-3-(cycloprop-2-en-1-yl)propanoat, Natrium-3-(cycloprop-1-en-1-yl)propanoat, Sidefungin, Spermidin, Spermine, Strigolactone, Tecnazene, Thidiazuron, Triacontanol, Trinexapac, Trinexapac-ethyl, Tryptophan, Tsitodef, Uniconazol, Uniconazol-P, 2-Fluoro-N-(3-methoxyphenyl)-*9H*-purin-6-amin.

Safener, die in Kombination mit den erfindungsgemäßen Verbindungen der Formel (I) und ggf. in Kombinationen mit weiteren Wirkstoffen wie z.B. Insektiziden, Akariziden, Herbiziden, Fungiziden wie oben aufgelistet, eingesetzt werden können, sind vorzugsweise ausgewählt aus der Gruppe bestehend aus:
S1) Verbindungen der Formel (S1), 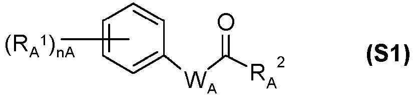 wobei die Symbole und Indizes folgende Bedeutungen haben:
   n_{A} ist eine natürliche Zahl von 0 bis 5, vorzugsweise 0 bis 3;
   R_{A}¹ ist Halogen, (C₁-C₄)Alkyl, (C₁-C₄)Alkoxy, Nitro oder (C₁-C₄)Haloalkyl;
   W_{A} ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴),
   m_{A} ist 0 oder 1; 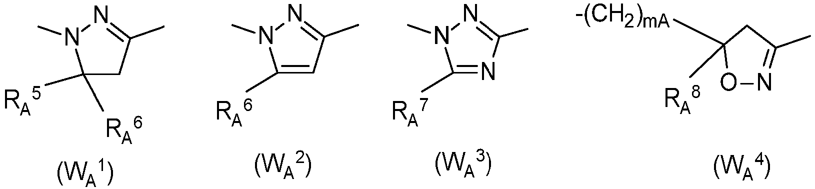
   R_{A}² ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   R_{A}³ ist Wasserstoff oder ein unsubstituierter oder substituierter aliphatischer Kohlenwasserstoffrest, vorzugsweise mit insgesamt 1 bis 18 C-Atomen;
   R_{A}⁴ ist Wasserstoff, (C₁-C₆)Alkyl, (C₁-C₆)Alkoxy oder substituiertes oder unsubstituiertes Phenyl;
   R_{A}⁵ ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   R_{A}⁶, R_{A}⁷, R_{A}⁸ sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
      a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
      b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
      c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
      d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäure-ethylester (S1-7), und verwandte Verbindungen wie sie in EP-A-174 562 und EP-A-346 620 beschrieben sind;
      e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-3-carbonsäure (S1-10) oder 5,5-Diphenyl-2-isoxazolin-3-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 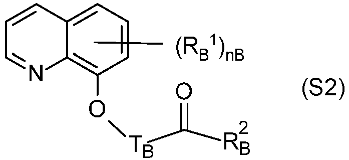 wobei die Symbole und Indizes folgende Bedeutungen haben:
   R_{B}¹ ist Halogen, (C₁-C₄)Alkyl, (C₁-C₄)Alkoxy, Nitro oder (C₁-C₄)Haloalkyl;
   n_{B} ist eine natürliche Zahl von 0 bis 5, vorzugsweise 0 bis 3;
   R_{B}² ist OR_{B}³, SR_{B}³ oder NR_{B}³R_{B}⁴ oder ein gesättigter
   oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³,
   NHR_{B}⁴ oder N(CH₃)₂, insbesondere der Formel OR_{B}³;
   R_{B}³ ist Wasserstoff oder ein unsubstituierter oder substituierter aliphatischer Kohlenwasserstoffrest, vorzugsweise mit insgesamt 1 bis 18 C-Atomen;
   R_{B}⁴ ist Wasserstoff, (C₁-C₆)Alkyl, (C₁-C₆)Alkoxy oder substituiertes oder unsubstituiertes Phenyl;
   T_{B} ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
      a) Verbindungen vom Typ der 8-Chinolinoxyessigsäure (S2^{a}), vorzugsweise
         (5-Chlor-8-chinolinoxy)essigsäure-(1-methylhexyl)ester ("Cloquintocet-mexyl") (S2-1),
         (5-Chlor-8-chinolinoxy)essigsäure-(1,3-dimethyl-but-1-yl)ester (S2-2),
         (5-Chlor-8-chinolinoxy)essigsäure-4-allyloxy-butylester (S2-3),
         (5-Chlor-8-chinolinoxy)essigsäure-1-allyloxy-prop-2-ylester (S2-4),
         (5-Chlor-8-chinolinoxy)essigsäureethylester (S2-5),
         (5-Chlor-8-chinolinoxy)essigsäuremethylester (S2-6),
         (5-Chlor-8-chinolinoxy)essigsäureallylester (S2-7),
         (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in
         EP-A-86 750, EP-A-94 349 und EP-A-191 736 oder EP-A-0 492 366 beschrieben sind, sowie (5-Chlor-
         8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
      b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 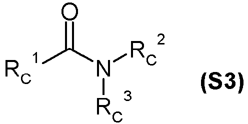 wobei die Symbole und Indizes folgende Bedeutungen haben:
   R_{C}¹ ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
      Wirkstoffe vom Typ der Dichloracetamide, die häufig als Vorauflaufsafener (bodenwirksame Safener) angewendet werden, wie z. B.
   "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4),
   "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw.
   Monsanto (S3-7),
   "TI-35" (1-Dichloracetyl-azepan) der Firma TRI-Chemical RT (S3-8),
   "Diclonon" (Dicyclonon) oder "BAS145138" oder "LAB145138" (S3-9)
   ((RS)-1-Dichloracetyl-3,3,8a-trimethylperhydropyrrolo[1,2-a]pyrimidin-6-on) der Firma BASF,
   "Furilazol" oder "MON 13900" ((RS)-3-Dichloracetyl-5-(2-furyl)-2,2-dimethyloxazolidin) (S3-10); sowie dessen (R)-Isomer (S3-11).
S4) N-Acylsulfonamide der Formel (S4) und ihre Salze, 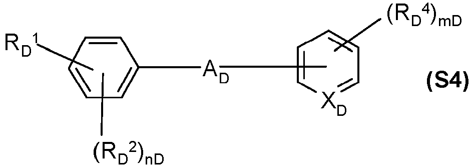 worin die Symbole und Indizes folgende Bedeutungen haben:
   A_{D} ist SO₂-NR_{D}³-CO oder CO-NR_{D}³-SO₂
   X_{D} ist CH oder N;
   R_{D}¹ ist CO-NR_{D}⁵R_{D}⁶ oder NHCO-R_{D}⁷;
   R_{D}² ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   R_{D}³ ist Wasserstoff, (C₁-C₄)Alkyl, (C₂-C₄)Alkenyl oder (C₂-C₄)Alkinyl,
   R_{D}⁴ ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   R_{D}⁵ ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
   R_{D}⁶ ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
   R_{D}⁵ und R_{D}⁶ gemeinsam mit dem dem sie tragenden Stickstoffatom einen Pyrrolidinyl- oder Piperidinyl-Rest bilden;
   R_{D}⁷ ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   n_{D} ist 0, 1 oder 2;
   m_{D} ist 1 oder 2;
   v_{D} ist 0, 1, 2 oder 3;
   davon bevorzugt sind Verbindungen vom Typ der N-Acylsulfonamide, z.B. der nachfolgenden Formel (S4^{a}), die z. B. bekannt sind aus WO-A-97/45016 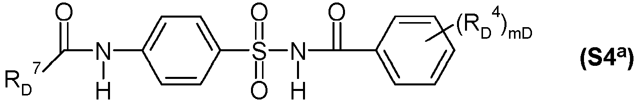
   worin
   R_{D}⁷ (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   R_{D}⁴ Halogen, (C₁-C₄)Alkyl, (C₁-C₄)Alkoxy, CF₃;
   m_{D} 1 oder 2;
   v_{D} ist 0, 1, 2 oder 3 bedeutet;
   sowie
   Acylsulfamoylbenzoesäureamide, z.B. der nachfolgenden Formel (S4^{b}), die z.B. bekannt sind aus WO-A-99/16744, 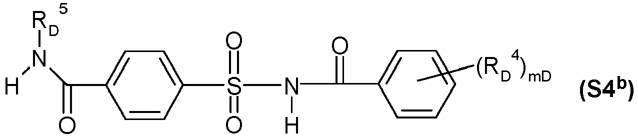
   z.B. solche worin
   R_{D}⁵ = Cyclopropyl und (R_{D}⁴) = 2-OMe ist ("Cyprosulfamide", S4-1),
   R_{D}⁵ = Cyclopropyl und (R_{D}⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵ = Isopropyl und (R_{D}⁴) = 2-OMe ist (S4-5).
   sowie
   Verbindungen vom Typ der N-Acylsulfamoylphenylharnstoffe der Formel (S4^{c}), die z.B. bekannt sind aus der EP-A-365484, 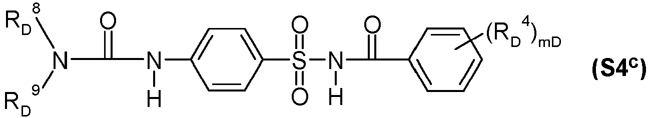
   worin
   R_{D}⁸ und R_{D}⁹ unabhängig voneinander Wasserstoff, (C₁-C₈)Alkyl, (C₃-C₈)Cycloalkyl, (C₃-C₆)Alkenyl, (C₃-C₆)Alkinyl,
   R_{D}⁴ Halogen, (C₁-C₄)Alkyl, (C₁-C₄)Alkoxy, CF₃
   m_{D} 1 oder 2 bedeutet;
   beispielsweise
   1-[4-(N-2-Methoxybenzoylsulfamoyl)phenyl]-3-methylharnstoff ("Metcamifen", S4-6),
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff,
   sowie
   N-Phenylsulfonylterephthalamide der Formel (S4^{d}), die z.B. bekannt sind aus CN 101838227, 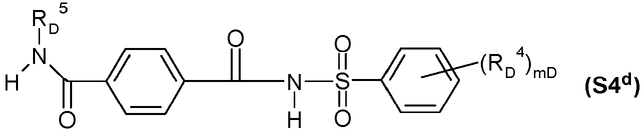
   z.B. solche worin
      - R_{D}⁴: Halogen, (C₁-C₄)Alkyl, (C₁-C₄)Alkoxy, CF_{3;}
      - m_{D}: 1 oder 2;
      - R_{D}⁵: Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl bedeutet.
S5) Wirkstoffe aus der Klasse der Hydroxyaromaten und der aromatisch-aliphatischen Carbonsäurederivate (S5), z.B.
   3,4,5-Triacetoxybenzoesäureethylester, 3,5-Dimethoxy-4-hydroxybenzoesäure, 3,5- Dihydroxybenzoesäure, 4-Hydroxysalicylsäure, 4-Fluorsalicyclsäure, 2-Hydroxyzimtsäure, 2,4-
   Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7), wie sie in der WO-A-1998/38856 beschrieben sind 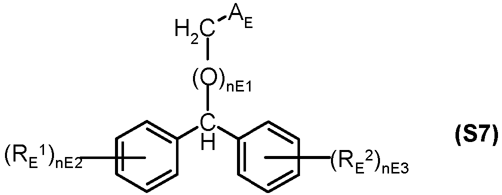 worin die Symbole und Indizes folgende Bedeutungen haben:
   R_{E}¹, R_{E}² sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   A_{E} ist COOR_{E}³ oder COSR_{E}⁴
   R_{E}³, R_{E}⁴ sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   n_{E}¹ ist 0 oder 1
   n_{E}², n_{E}³ sind unabhängig voneinander 0, 1 oder 2,
   vorzugsweise:
      Diphenylmethoxyessigsäure,
      Diphenylmethoxyessigsäureethylester,
      Diphenylmethoxyessigsäuremethylester (CAS-Reg.Nr. 41858-19-9) (S7-1).
S8) Verbindungen der Formel (S8),wie sie in der WO-A-98/27049 beschrieben sind worin 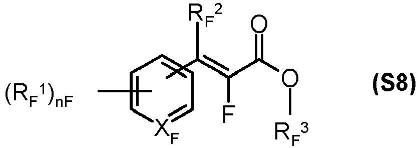
   X_{F} CH oder N,
   n_{F} für den Fall, dass X_{F}=N ist, eine ganze Zahl von 0 bis 4 und
      für den Fall, dass X_{F}=CH ist, eine ganze Zahl von 0 bis 5 ,
   R_{F}¹ Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   R_{F}² Wasserstoff oder (C₁-C₄)Alkyl
   R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; bedeuten, oder deren Salze,
   vorzugsweise Verbindungen worin
   X_{F} CH,
   n_{F} eine ganze Zahl von 0 bis 2 ,
   R_{F}¹ Halogen, (C₁-C₄)Alkyl, (C₁-C₄)Haloalkyl, (C₁-C₄)Alkoxy, (C₁-C₄)Haloalkoxy,
   R_{F}² Wasserstoff oder (C₁-C₄)Alkyl,
   R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten,
   oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10⁶)
   wie sie in der WO-A-2007/023719 und WO-A-2007/023764 beschrieben sind 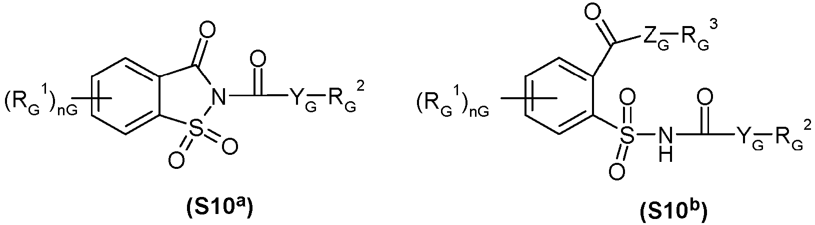 worin
   - R_{G}¹: Halogen, (C₁-C₄)Alkyl, Methoxy, Nitro, Cyano, CF₃, OCF₃
   - Y_{G}, Z_{G}: unabhängig voneinander O oder S,
   - n_{G}: eine ganze Zahl von 0 bis 4,
   - R_{G}²: (C₁-C₁₆)Alkyl, (C₂-C₆)Alkenyl, (C₃-C₆)Cycloalkyl, Aryl; Benzyl, Halogenbenzyl,
   - R_{G}³: Wasserstoff oder (C₁-C₆)Alkyl bedeutet.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für
   Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG 838" (CAS-Reg.Nr. 133993-74-5) (2-propenyl 1-oxa-4-azaspiro[4.5]decan-4-carbodithioat) (S13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorothioat) (S13-8),
   "Mephenate" (4-Chlorphenyl-methylcarbamat) (S13-9).
   S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY 93" (S-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC 940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere 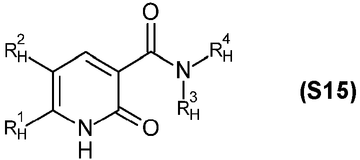
   wie sie in der WO-A-2008/131861 und WO-A-2008/131860 beschrieben sind
      worin
      - R_{H}¹: einen (C₁-C₆)Haloalkylrest bedeutet und
      - R_{H}²: Wasserstoff oder Halogen bedeutet und
      - R_{H}³, R_{H}⁴: unabhängig voneinander Wasserstoff, (C₁-C₁₆)Alkyl, (C₂-C₁₆)Alkenyl oder (C₂-C₁₆)Alkinyl,
   wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
   wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C_{1-C4})Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   bedeutet oder
   R_{H}³ (C₁-C₄)-Alkoxy, (C₂-C₄)Alkenyloxy, (C₂-C₆)Alkinyloxy oder (C₂-C₄)Haloalkoxy bedeutet und
   R_{H}⁴ Wasserstoff oder (C₁-C₄)-Alkyl bedeutet oder
   R_{H}³ und R_{H}⁴ zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Besonders bevorzugte Safener sind Mefenpyr-diethyl, Cyprosulfamid, Isoxadifen-ethyl, Cloquintocet-mexyl, Benoxacor, Dichlormid und Metcamifen.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolether-sulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepoly-glykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfett-säureester oder Polyoxethylensorbitanester wie z.B. Polyoxyethylensorbitan-fettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoff-konzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasser-dispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I) und deren Salze. Sie kann innerhalb weiter Grenzen schwanken, z.B. zwischen 0,001 und 10,0 kg/ha oder mehr Aktivsubstanz, vorzugsweise liegt sie jedoch zwischen 0,005 bis 5 kg/ha, weiter bevorzugt im Bereich von 0,01 bis 1,5 kg/ha, insbesondere bevorzugt im Bereich von 0,05 bis 1 kg/ha g/ha. Dies gilt sowohl für die Anwendung im Vorauflauf oder im Nachauflauf.

Trägerstoff bedeutet eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein. Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels. Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden. Im Allgemeinen enthalten die erfindungsgemäßen Mittel und Formulierungen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent. Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgut-behandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen. Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende Hauptanbaupflanzen erwähnt: Mais, Sojabohne, Baumwolle, Brassica Ölsaaten wie Brassica napus (z.B. Canola), Brassica rapa, B. juncea (z.B. (Acker-)Senf) und Brassica carinata, Reis, Weizen Zuckerrübe, Zurckerrohr, Hafer, Roggen, Gerste, Hirse, Triticale, Flachs, Wein und verschiedene Früchte und Gemüse von verschiedenen botanischen Taxa wie z.B. Rosaceae sp. (beispielsweise Kernfrüchte wie Apfel und Birne, aber auch Steinfrüchte wie Aprikosen, Kirschen, Mandeln und Pfirsiche und Beerenfrüchte wie Erdbeeren), Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp. (beispielsweise Bananenbäume und -plantagen), Rubiaceae sp. (beispielsweise Kaffee), Theaceae sp., Sterculiceae sp., Rutaceae sp. (beispielsweise Zitronen, Organen und Grapefruit); Solanaceae sp. (beispielsweise Tomaten, Kartoffeln, Pfeffer, Auberginen), Liliaceae sp., Compositae sp. (beispielsweise Salat, Artischocke and Chicoree - einschließlich Wurzelchicoree, Endivie oder gemeinen Chicoree), Umbelliferae sp. (beispielsweise Karrotte, Petersilie, Stangensellerie und Knollensellerie), Cucurbitaceae sp. (beispielsweise Gurke - einschließlich Gewürzgurke, Kürbis, Wassermelone, Flaschenkürbis und Melonen), Alliaceae sp. (beispielsweise Lauch und Zwiebel), Cruciferae sp. (beispielsweise Weißkohl, Rotkohl, Brokkoli, Blumenkohl, Rosenkohl, Pak Choi, Kohlrabi, Radieschen, Meerrettich, Kresse und Chinakohl), Leguminosae sp. (beispielsweise Erdnüsse, Erbsen, und Bohnen - wie z.B. Stangenbohne und Ackerbohne), Chenopodiaceae sp. (beispielsweise Mangold, Futterrübe, Spinat, Rote Rübe), Malvaceae (beispielsweise Okra), Asparagaceae (beispielsweise Spargel); Nutzpflanzen und Zierpflanzen in Garten und Wald; sowie jeweils genetisch modifizierte Arten dieser Pflanzen.

Wie oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. Pflanzen können mit verschiedenen Methoden tolerant gegenüber Glyphosate gemacht werden. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakteriums Salmonella typhimurium (Comai et al., 1983, Science 221, 370-371), das CP4-Gen des Bakteriums Agrobacterium sp. (Barry et al., 1992, Curr. Topics Plant Physiol. 7, 139-145), die Gene, die für eine EPSPS aus der Petunie (Shah et al., 1986, Science 233, 478-481), für eine EPSPS aus der Tomate (Gasser et al., 1988, J. Biol. Chem. 263, 4280-4289) oder für eine EPSPS aus Eleusine (WO 01/66704) kodieren. Es kann sich auch um eine mutierte EPSPS handeln. Glyphosate-tolerante Pflanzen können auch dadurch erhalten werden, dass man ein Gen exprimiert, das für ein Glyphosate-Oxidoreduktase-Enzym kodiert. Glyphosate-tolerante Pflanzen können auch dadurch erhalten werden, dass man ein Gen exprimiert, das für ein Glyphosateacetyltransferase-Enzym kodiert. Glyphosatetolerante Pflanzen können auch dadurch erhalten werden, dass man Pflanzen, die natürlich vorkommende Mutationen der oben erwähnten Gene enthalten, selektiert. Pflanzen, die EPSPS Gene, welche Glyphosate-Toleranz verleihen, exprimieren, sind beschrieben. Pflanzen, welche andere Gene, die Glyphosate-Toleranz verleihen, z.B. Decarboxylase-Gene, sind beschrieben.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben. Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes oder chimäres HPPD-Enzym kodiert, transformiert werden, wie in WO 96/38567, WO 99/24585, WO 99/24586, WO 2009/144079, WO 2002/046387 oder US 6,768,044 beschrieben. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen sind in WO 99/34008 und WO 02/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert, wie in WO 2004/024928 beschrieben ist. Außerdem können Pflanzen noch toleranter gegen HPPD-Hemmern gemacht werden, indem man ein Gen in ihr Genom einfügt, welches für ein Enzym kodiert, das HPPD-Hemmer metabolisiert oder abbaut, wie z.B. CYP450 Enzyme (siehe WO 2007/103567 und WO 2008/150473).

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen wie z.B. in Tranel und Wright (Weed Science 2002, 50, 700-712) beschrieben ist. Die Herstellung von sulfonylharnstofftoleranten Pflanzen und imidazolinontoleranten Pflanzen ist beschrieben. Weitere sulfonylharnstoff- und imidazolinontolerante Pflanzen sind auch beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden (vgl. z.B. für Sojabohne US 5,084,082, für Reis WO 97/41218, für Zuckerrübe US 5,773,702 und WO 99/057965, für Salat US 5,198,599 oder für Sonnenblume WO 01/065922).

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst im vorliegenden Zusammenhang jegliche Pflanze, die mindestens ein Transgen enthält, das eine Kodiersequenz umfasst, die für folgendes kodiert:
1) ein insektizides Kristallprotein aus Bacillus thuringiensis oder einen insektiziden Teil davon, wie die insektiziden Kristallproteine, aufgelistet von Crickmore et al. (Microbiology and Molecular Biology Reviews 1998, 62, 807-813), aktualisiert von Crickmore et al. (2005) bei der Bacillus thuringiensis Toxin Nomenclatur, online bei: http://www.lifesci.sussex.ac.uk/Home/Neil_Crickmore/Bt/), oder insektizide Teile davon, z.B. Proteine der Cry-Proteinklassen Cry1Ab, Cry1Ac, Cry1B, Cry1C, Cry1D, Cry1F, Cry2Ab, Cry3Aa, or Cry3Bb oder insektizide Teile davon (z.B. EP-A 1999141 und WO 2007/107302), oder solche Proteine, kodiert durch synthetische Gene wie in US Patentanmeldung 12/249,016 beschrieben ist; oder
2) ein Kristallprotein aus Bacillus thuringiensis oder einen Teil davon, der in Gegenwart eines zweiten, anderen Kristallproteins als Bacillus thuringiensis oder eines Teils davon insektizid wirkt, wie das binäre Toxin, das aus den Kristallproteinen Cy34 und Cy35 besteht (Nat. Biotechnol. 2001, 19, 668-72; Applied Environm. Microbiol. 2006, 71, 1765-1774) oder das binäre Toxin, das aus den Cry1A oder Cry1F Proteinen besteht und die Cry2Aa oder Cry2Ab oder Cry2Ae Proteine (US Patentanmeldung 12/214,022 und EP08010791.5); oder
3) ein insektizides Hybridprotein, das Teile von zwei unterschiedlichen insektiziden Kristallproteinen aus Bacillus thuringiensis umfasst, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus Bacillus thuringiensis oder Bacillus cereus oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus Bacillus thuringiensis oder Bacillus cereus, das in Gegenwart eines zweiten sezernierten Proteins aus Bacillus thuringiensis oder B. cereus insektizid wirkt, wie das binäre Toxin, das aus den Proteinen VIP1A und VIP2A besteht (WO 94/21795); oder
7) ein insektizides Hybridprotein, das Teile von verschiedenen sezernierten Proteinen von Bacillus thuringiensis oder Bacillus cereus umfasst, wie ein Hybrid der Proteine von 1) oder ein Hybrid der Proteine von 2) oben; oder
8) ein Protein gemäß einem der Punkte 5) bis 7) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102; oder
9) ein sezerniertes Protein aus Bacillus thuringiensis oder Bacillus cereus, das in Gegenwart eines Kristallproteins von Bacillus thuringiensis insektizid wirkt, wie das binäre Toxin, das aus den Proteinen VIP3 und Cry1A oder Cry1F besteht (US Patentanmeldungen 61/126083 und 61/195019), oder das binäre Toxin, das aus dem VIP3 Protein und den Cry2Aa oder Cry2Ab oder Cry2Ae Proteinen besteht (US Patentanmeldung 12/214,022 und EP 08010791.5); oder
10) ein Protein gemäß Punkt 9) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt). Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 10 kodieren. In einer Ausführungsform enthält eine insektenresistente Pflanze mehr als ein Transgen, das für ein Protein nach einer der oben genannten 1 bis 10 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Eine "insekten-resistente transgene Pflanze" umfasst im vorliegenden Zusammenhang weiterhin jede Pflanze, die wenigstens ein Transgen enthält, welches eine Sequenz zur Herstellung einer Doppelstrang-RNA umfasst, die nach Nahrungsaufnahme durch einen Insektenschädling das Wachstum dieses Schädlings hindert.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.
4) Transgene Pflanzen oder Hybridpflanzen wie Zwiebeln mit bestimmten Eigenschaften wie "hohem Anteil an löslichen Feststoffen" (,high soluble solids content'), geringe Schärfe (,low pungency', LP) und/oder lange Lagerfähigkeit (,long storage', LS).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten, wie Baumwollpflanzen mit einer erhöhten Expression der Saccharosephosphatsynthase;
c) Pflanzen wie Baumwollpflanzen mit einer erhöhten Expression der Saccharosesynthase;
d) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
e) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Pflanzen oder Pflanzensorten (die nach Methoden der pflanzlichen Biotechnologie, wie der Gentechnik, erhalten werden können), die ebenfalls erfindungsgemäß behandelt werden können, sind Pflanzen wie Kartoffeln, welche Virus-resistent sind z.B. gegen den Kartoffelvirus Y (Event SY230 und SY233 von Tecnoplant, Argentinien), oder welche resistent gegen Krankheiten wie die Kraut- und Knollenfäule (potato late blight) (z.B. RB Gen), oder welche eine verminderte kälteinduzierte Süße zeigen (welche die Gene Nt-Inh, II-INV tragen) oder welche den Zwerg-Phänotyp zeigen (Gen A-20 Oxidase).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften im Samenausfall (seed shattering). Solche Pflanzen können durch genetische Transformation oder durch Selektion von Pflanzen, die eine Mutation enthalten, die solche veränderten Eigenschaften verleihen, und umfassen Pflanzen wie Raps mit verzögertem oder vermindertem Samenausfall.

Besonders nützliche transgene Pflanzen, die erfindungsgemäß behandelt werden können, sind Pflanzen mit Transformationsevents oder Kombinationen von Transformationsevent, welche in den USA beim Animal and Plant Health Inspection Service (APHIS) of the United States Department of Agriculture (USDA) Gegenstand von erteilten oder anhängigen Petitionen für den nicht-regulierten Status sind. Die Information hierzu ist jederzeit beim APHIS (4700 River Road Riverdale, MD 20737, USA) erhältlich, z.B. über die Internetseite http://www.aphis.usda.gov/brs/not_reg.html. Am Anmeldetag dieser Anmeldung waren beim APHIS die Petitionen mit folgenden Informationen entweder erteilt oder anhängig:
- Petition: Identifikationsnummer der Petition. Die Technische Beschreibung des Transformations-events kann im einzelnen Petitionsdokument erhältlich von APHIS auf der Website über die Petitionsnummer gefunden werden. Diese Beschreibungen sind hiermit per Referenz offenbart.
- Erweiterung einer Petition: Referenz zu einer frühere Petition, für die eine Erweiterung oder Verlängerung beantragt wird.
- Institution: Name der die Petition einreichenden Person.
- Regulierter Artikel: die betroffen Pflanzenspecies.
- Transgener Phänotyp: die Eigenschaft ("Trait"), die der Pflanze durch das Transformationsevent verliehen wird.
- Transformationevent oder -linie: der Name des oder der Events (manchmal auch als Linie(n) bezeichnet), für die der nicht-regulierte Status beantragt ist.
- APHIS Documente: verschiedene Dokumente, die von APHIS bzgl. der Petition veröffentlicht warden oder von APHIS auf Anfrage erhalten werden können.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD^{®} (zum Beispiel Mais, Baumwolle, Sojabohnen), KnockOut^{®} (zum Beispiel Mais), BiteGard^{®} (zum Beispiel Mais), BT-Xtra^{®} (zum Beispiel Mais), StarLink^{®} (zum Beispiel Mais), Bollgard^{®} (Baumwolle), Nucotn^{®} (Baumwolle), Nucotn 33B^{®} (Baumwolle), NatureGard^{®} (zum Beispiel Mais), Protecta^{®} und NewLeaf^{®} (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready^{®} (Glyphosatetoleranz, zum Beispiel Mais, Baumwolle, Sojabohne), Liberty Link^{®} (Phosphinotricintoleranz, zum Beispiel Raps), IMI^{®} (Imidazolinontoleranz) und SCS^{®} (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield^{®} angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfojrc.it/gmp_browse.aspx und http://cera-gmc.org/index.php?evidcode=&hstIDXCode=&gType=&AbbrCode=&atCode=&stCode=&coID Code=&action=gm_crop_database&mode=Submit).

### A. Chemische Beispiele

Die nachfolgenden Beispiele erläutern die vorliegende Erfindung.

### Synthese von 2-Chlor-3-(methylsulfanyl)-N-(1,3,4-oxadiazol-2-yl)-4-(trifluormethoxy)benzamid (Beispiel-Nr. 1-31)

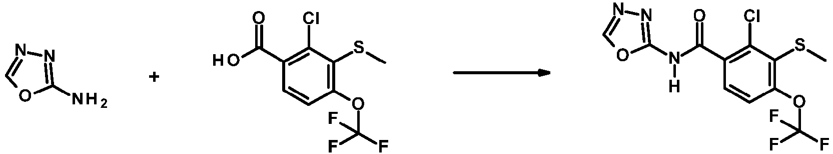

653 mg (7.68 mmol) 1,3,4-Oxadiazol-2-amin und 2.00 g (6.98 mmol) 2-Chlor-3-(methylsulfanyl)-4-(trifluormethoxy)benzoesäure wurden in 50 ml Acetonitril mit 2.67 ml (33.49 mmol) 1-Methyl-1H-imidazol versetzt. Das Gemisch wurde auf eine Temperatur von 0 °C - 5 °C abgekühlt. 0.91 ml (10.47 mmol) Oxalsäuredichlorid wurden portionsweise zugegeben. Anschließend wurde das Reaktionsgemisch auf Raumtemperatur erwärmt und 3.5 h bei dieser Temperatur gerührt. Zur Aufarbeitung wurde das Reaktionsgemisch am Rotationsverdampfer vom Lösungsmittel befreit und der Rückstand wurde in Dichlormethan und Wasser aufgenommen. Nach der Phasentrennung wurde die organische Phase eingeengt und der Rückstand wurde in Wasser aufgenommen. Es wurde 6 M Natronlauge zugegeben, anschließend wurde mehrmals mit Dichlormethan gewaschen. Im Anschluss wurde die wässrige Phase mit 6 M Salzsäure angesäuert. Der entstandene Feststoff wurde abfiltriert und getrocknet. Danach wurde der Feststoff nochmals in Dichlormethan und einer wässrigen Lösung von Natriumhydrogencarbonat aufgenommen. Nach der Phasentrennung wurde die organische Phase am Rotationsverdampfer vom Lösungsmittel befreit. Es wurden 910 mg des gewünschten Produkts mit einer Reinheit von 85 Gew.-% isoliert.

### Synthese von 2-Chlor-3-(methylsulfinyl)-N-(1,3,4-oxadiazol-2-yl)-4-(trifluormethoxy)benzamid (Beispiel-Nr. 1-32) und 2-Chlor-3-(methylsulfonyl)-N-(1,3,4-oxadiazol-2-yl)-4-(trifluormethoxy)benzamid (Beispiel-Nr. 1-35)

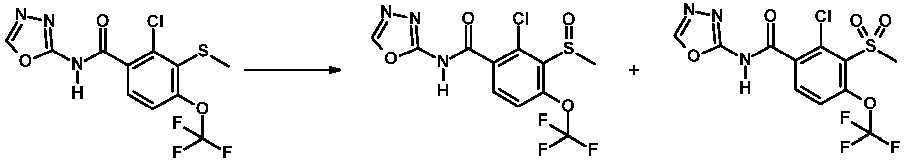

750 mg (85 Gew.-%; 1.80 mmol) 2-Chlor-3-(methylsulfanyl)-N-(1,3,4-oxadiazol-2-yl)-4-(trifluormethoxy)benzamid wurden in 25 ml Dichlormethan bei Raumtemperatur mit 713 mg (77 Gew.-%; 3.18 mmol) 3-Chlorperoxybenzoesäure versetzt. Das Gemisch wurde 6 d bei Raumtemperatur gerührt. Anschließend wurden weitere 119 mg (77 Gew.-%; 0.53 mmol) 3-Chlorperoxybenzoesäure zugegeben und der Ansatz wurde so lange bei Raumtemperatur gerührt, bis die Reaktionskontrolle neben dem Sulfoxid auch eine signifikante Menge Sulfon anzeigte. Zur Aufarbeitung wurde eine wässrige Lösung von Natriummetabisulfit zugegeben. Das Gemisch wurde einige Minuten gerührt und nach der Phasentrennung wurde die organische Phase am Rotationsverdampfer vom Lösungsmittel befreit. Der Rückstand wurde chromatographisch gereinigt, wobei 112 mg 2-Chlor-3-(methylsulfinyl)-N-(1,3,4-oxadiazol-2-yl)-4-(trifluormethoxy)benzamid mit einer Reinheit von 90 Gew.-% und 68 mg 2-Chlor-3-(methylsulfonyl)-N-(1,3,4-oxadiazol-2-yl)-4-(trifluormethoxy)benzamid mit einer Reinheit von 90 Gew.-% gewonnen wurden.

### Synthese von 2-Chlor-4-(difluormethoxy)-3-(methylsulfanyl)-N-(1,3,4-oxadiazol-2-yl)benzamid (Beispiel-Nr. 1-21)

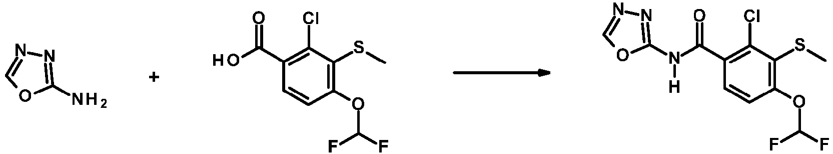

697 mg (8.19 mmol) 1,3,4-Oxadiazol-2-amin und 2.00 g (7.44 mmol) 2-Chlor-4-(difluormethoxy)-3-(methylsulfanyl)benzoesäure wurden in 50 ml Acetonitril mit 2.85 ml (35.73 mmol) 1-Methyl-1H-imidazol versetzt. Das Gemisch wurde auf eine Temperatur von 0 °C - 5 °C abgekühlt. 0.97 ml (11.17 mmol) Oxalsäuredichlorid wurden portionsweise zugegeben. Anschließend wurde das Reaktionsgemisch auf Raumtemperatur erwärmt und 16 h bei dieser Temperatur gerührt. Zur Aufarbeitung wurde das Reaktionsgemisch am Rotationsverdampfer vom Lösungsmittel befreit und der Rückstand wurde in Dichlormethan und Wasser aufgenommen. Nach der Phasentrennung wurde die organische Phase eingeengt und der Rückstand wurde in Wasser aufgenommen. Es wurde 6 M Natronlauge zugegeben, anschließend wurde mehrmals mit Dichlormethan gewaschen. Im Anschluss wurde die wässrige Phase mit 6 M Salzsäure angesäuert. Das Gemisch wurde filtriert und der entstandene Feststoff wurde getrocknet. Danach wurde der Feststoff nochmals in Dichlormethan und einer wässrigen Lösung von Natriumhydrogencarbonat aufgenommen. Nach der Phasentrennung wurde die organische Phase am Rotationsverdampfer vom Lösungsmittel befreit. Der Rückstand wurde mit wenig Dichlormethan gerührt, anschließend wurde das Gemisch filtriert. Der Feststoff wurde getrocknet und es wurden 605 mg des gewünschten Produkts mit einer Reinheit von 80 Gew.-% isoliert.

### Synthese von 2-Chlor-4-(difluormethoxy)-3-(methylsulfonyl)-N-(1,3,4-oxadiazol-2-yl)benzamid (Beispiel-Nr. 1-25)

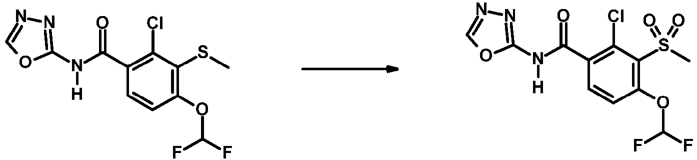

600 mg (80 Gew.-%; 1.43 mmol) 2-Chlor-4-(difluormethoxy)-3-(methylsulfanyl)-N-(1,3,4-oxadiazol-2-yl)benzamid wurden in 36 ml Dichlormethan bei Raumtemperatur mit 1001 mg (77 Gew.-%; 4.47 mmol) 3-Chlorperoxybenzoesäure versetzt. Das Gemisch wurde 7 d bei Raumtemperatur gerührt. Anschließend wurden zur Vervollständigung der Reaktion zweimal jeweils 123 mg (77 Gew.-%; 0.55 mmol) 3-Chlorperoxybenzoesäure sowie 5 ml Acetonitril zugegeben. Der Ansatz wurde so lange bei Raumtemperatur gerührt, bis die Reaktionskontrolle die fertige Umsetzung zum Sulfon anzeigte. Zur Aufarbeitung wurde eine wässrige Lösung von Natriummetabisulfit zugegeben. Das Gemisch wurde einige Minuten gerührt und nach der Phasentrennung wurde die organische Phase am Rotationsverdampfer vom Lösungsmittel befreit. Der Rückstand wurde in tert-Butylmethylether aufgenommen, anschließend wurde das Gemisch filtriert und der isolierte Feststoff wurde getrocknet. Als Feststoff wurden 427 mg des gewünschten Produkts mit einer Reinheit von 90 Gew.-% isoliert.

Die in nachfolgenden Tabellen aufgeführten Beispiele wurden analog oben genannten Methoden hergestellt beziehungsweise sind analog oben genannten Methoden erhältlich. Diese Verbindungen sind ganz besonders bevorzugt.

Die hier verwendeten Abkürzungen bedeuten:

| | | | | | |
|---|---|---|---|---|---|
| Me | = Methyl | Et | = Ethyl | MeO | = Methoxy |
| EtO | = Ethoxy | MeOCH₂ | = Methoxymethyl | MeS | = Methylthio |
| HF₂CO | = Difluormethoxy | F₃CO | = Trifluormethoxy | | |

**Tabelle 1: Erfindungsgemäße Verbindungen der allgemeinen Formel (I), worin die Substituenten die unten genannten Bedeutungen haben und Verbindungen mit X=F nicht Gegenstand der Erfindung sind. Im Fall von "n = 1" liegen chirale Sulfoxide vor. Hierfür wird in der Spalte "Sulfoxid-Konfiguration" angegeben, ob das Sulfoxid in der (R)-Konfiguration, in der (S)-Konfiguration oder ob es racemisch in beiden Konfigurationen vorliegt.**

| 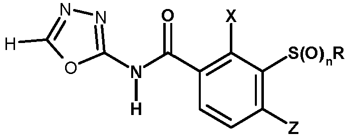 | | | | | |
|---|---|---|---|---|---|
| Nr. | X | Z | n | Sulfoxid-Konfiguration | R |
| 1-1 | F | HF₂CO | 0 | | Me |
| 1-2 | F | HF₂CO | 1 | racemisch | Me |
| 1-3 | F | HF₂CO | 1 | *(R)* | Me |
| 1-4 | F | HF₂CO | 1 | (S) | Me |
| 1-5 | F | HF₂CO | 2 | | Me |
| 1-6 | F | HF₂CO | 0 | | Et |
| 1-7 | F | HF₂CO | 1 | racemisch | Et |
| 1-8 | F | HF₂CO | 1 | *(R)* | Et |
| 1-9 | F | HF₂CO | 1 | (*S*) | Et |
| 1-10 | F | HF₂CO | 2 | | Et |
| 1-11 | F | F₃CO | 0 | | Me |
| 1-12 | F | F₃CO | 1 | racemisch | Me |
| 1-13 | F | F₃CO | 1 | *(R)* | Me |
| 1-14 | F | F₃CO | 1 | (*S*) | Me |
| 1-15 | F | F₃CO | 2 | | Me |
| 1-16 | F | F₃CO | 0 | | Et |
| 1-17 | F | F₃CO | 1 | racemisch | Et |
| 1-18 | F | F₃CO | 1 | *(R)* | Et |
| 1-19 | F | F₃CO | 1 | (*S*) | Et |
| 1-20 | F | F₃CO | 2 | | Et |
| 1-21 | Cl | HF₂CO | 0 | | Me |
| 1-22 | Cl | HF₂CO | 1 | racemisch | Me |
| 1-23 | Cl | HF₂CO | 1 | *(R)* | Me |
| 1-24 | Cl | HF₂CO | 1 | (*S*) | Me |
| 1-25 | Cl | HF₂CO | 2 | | Me |
| 1-26 | Cl | HF₂CO | 0 | | Et |
| 1-27 | Cl | HF₂CO | 1 | racemisch | Et |
| 1-28 | Cl | HF₂CO | 1 | *(R)* | Et |
| 1-29 | Cl | HF₂CO | 1 | (*S*) | Et |
| 1-30 | Cl | HF₂CO | 2 | | Et |
| 1-31 | Cl | F₃CO | 0 | | Me |
| 1-32 | Cl | F₃CO | 1 | racemisch | Me |
| 1-33 | Cl | F₃CO | 1 | *(R)* | Me |
| 1-34 | Cl | F₃CO | 1 | (*S*) | Me |
| 1-35 | Cl | F₃CO | 2 | | Me |
| 1-36 | Cl | F₃CO | 0 | | Et |
| 1-37 | Cl | F₃CO | 1 | racemisch | Et |
| 1-38 | Cl | F₃CO | 1 | *(R)* | Et |
| 1-39 | Cl | F₃CO | 1 | (*S*) | Et |
| 1-40 | Cl | F₃CO | 2 | | Et |
| 1-41 | Br | HF₂CO | 0 | | Me |
| 1-42 | Br | HF₂CO | 1 | racemisch | Me |
| 1-43 | Br | HF₂CO | 1 | *(R)* | Me |
| 1-44 | Br | HF₂CO | 1 | (*S*) | Me |
| 1-45 | Br | HF₂CO | 2 | | Me |
| 1-46 | Br | HF₂CO | 0 | | Et |
| 1-47 | Br | HF₂CO | 1 | racemisch | Et |
| 1-48 | Br | HF₂CO | 1 | *(R)* | Et |
| 1-49 | Br | HF₂CO | 1 | (*S*) | Et |
| 1-50 | Br | HF₂CO | 2 | | Et |
| 1-51 | Br | F₃CO | 0 | | Me |
| 1-52 | Br | F₃CO | 1 | racemisch | Me |
| 1-53 | Br | F₃CO | 1 | *(R)* | Me |
| 1-54 | Br | F₃CO | 1 | (*S*) | Me |
| *1-55* | Br | F₃CO | 2 | | Me |
| 1-56 | Br | F₃CO | 0 | | Et |
| 1-57 | Br | F₃CO | 1 | racemisch | Et |
| 1-58 | Br | F₃CO | 1 | *(R)* | Et |
| 1-59 | Br | F₃CO | 1 | (*S*) | Et |
| 1-60 | Br | F₃CO | 2 | | Et |
| 1-61 | Me | HF₂CO | 0 | | Me |
| 1-62 | Me | HF₂CO | 1 | racemisch | Me |
| 1-63 | Me | HF₂CO | 1 | *(R)* | Me |
| 1-64 | Me | HF₂CO | 1 | (*S*) | Me |
| 1-65 | Me | HF₂CO | 2 | | Me |
| 1-66 | Me | HF₂CO | 0 | | Et |
| 1-67 | Me | HF₂CO | 1 | racemisch | Et |
| 1-68 | Me | HF₂CO | 1 | *(R)* | Et |
| 1-69 | Me | HF₂CO | 1 | (*S*) | Et |
| 1-70 | Me | HF₂CO | 2 | | Et |
| 1-71 | Me | F₃CO | 0 | | Me |
| 1-72 | Me | F₃CO | 1 | racemisch | Me |
| 1-73 | Me | F₃CO | 1 | *(R)* | Me |
| 1-74 | Me | F₃CO | 1 | (*S*) | Me |
| 1-75 | Me | F₃CO | 2 | | Me |
| 1-76 | Me | F₃CO | 0 | | Et |
| 1-77 | Me | F₃CO | 1 | racemisch | Et |
| 1-78 | Me | F₃CO | 1 | *(R)* | Et |
| 1-79 | Me | F₃CO | 1 | (*S*) | Et |
| 1-80 | Me | F₃CO | 2 | | Et |
| 1-81 | Et | HF₂CO | 0 | | Me |
| 1-82 | Et | HF₂CO | 1 | racemisch | Me |
| 1-83 | Et | HF₂CO | 1 | *(R)* | Me |
| 1-84 | Et | HF₂CO | 1 | (*S*) | Me |
| 1-85 | Et | HF₂CO | 2 | | Me |
| 1-86 | Et | HF₂CO | 0 | | Et |
| 1-87 | Et | HF₂CO | 1 | racemisch | Et |
| 1-88 | Et | HF₂CO | 1 | *(R)* | Et |
| 1-89 | Et | HF₂CO | 1 | (*S*) | Et |
| 1-90 | Et | HF₂CO | 2 | | Et |
| 1-91 | Et | F₃CO | 0 | | Me |
| 1-92 | Et | F₃CO | 1 | racemisch | Me |
| 1-93 | Et | F₃CO | 1 | *(R)* | Me |
| 1-94 | Et | F₃CO | 1 | (*S*) | Me |
| 1-95 | Et | F₃CO | 2 | | Me |
| 1-96 | Et | F₃CO | 0 | | Et |
| 1-97 | Et | F₃CO | 1 | racemisch | Et |
| 1-98 | Et | F₃CO | 1 | *(R)* | Et |
| 1-99 | Et | F₃CO | 1 | (*S*) | Et |
| 1-100 | Et | F₃CO | 2 | | Et |
| 1-101 | MeO | HF₂CO | 0 | | Me |
| 1-102 | MeO | HF₂CO | 1 | racemisch | Me |
| 1-103 | MeO | HF₂CO | 1 | *(R)* | Me |
| 1-104 | MeO | HF₂CO | 1 | (*S*) | Me |
| 1-105 | MeO | HF₂CO | 2 | | Me |
| 1-106 | MeO | HF₂CO | 0 | | Et |
| 1-107 | MeO | HF₂CO | 1 | racemisch | Et |
| 1-108 | MeO | HF₂CO | 1 | *(R)* | Et |
| 1-109 | MeO | HF₂CO | 1 | (*S*) | Et |
| 1-110 | MeO | HF₂CO | 2 | | Et |
| 1-111 | MeO | F₃CO | 0 | | Me |
| 1-112 | MeO | F₃CO | 1 | racemisch | Me |
| 1-113 | MeO | F₃CO | 1 | *(R)* | Me |
| 1-114 | MeO | F₃CO | 1 | (*S*) | Me |
| 1-115 | MeO | F₃CO | 2 | | Me |
| 1-116 | MeO | F₃CO | 0 | | Et |
| 1-117 | MeO | F₃CO | 1 | racemisch | Et |
| 1-118 | MeO | F₃CO | 1 | *(R)* | Et |
| 1-119 | MeO | F₃CO | 1 | (*S*) | Et |
| 1-120 | MeO | F₃CO | 2 | | Et |
| 1-121 | EtO | HF₂CO | 0 | | Me |
| 1-122 | EtO | HF₂CO | 1 | racemisch | Me |
| 1-123 | EtO | HF₂CO | 1 | *(R)* | Me |
| 1-124 | EtO | HF₂CO | 1 | (*S*) | Me |
| 1-125 | EtO | HF₂CO | 2 | | Me |
| 1-126 | EtO | HF₂CO | 0 | | Et |
| 1-127 | EtO | HF₂CO | 1 | racemisch | Et |
| 1-128 | EtO | HF₂CO | 1 | *(R)* | Et |
| 1-129 | EtO | HF₂CO | 1 | (*S*) | Et |
| 1-130 | EtO | HF₂CO | 2 | | Et |
| 1-131 | EtO | F₃CO | 0 | | Me |
| 1-132 | EtO | F₃CO | 1 | racemisch | Me |
| 1-133 | EtO | F₃CO | 1 | *(R)* | Me |
| 1-134 | EtO | F₃CO | 1 | (*S*) | Me |
| 1-135 | EtO | F₃CO | 2 | | Me |
| 1-136 | EtO | F₃CO | 0 | | Et |
| 1-137 | EtO | F₃CO | 1 | racemisch | Et |
| 1-138 | EtO | F₃CO | 1 | *(R)* | Et |
| 1-139 | EtO | F₃CO | 1 | (*S*) | Et |
| 1-140 | EtO | F₃CO | 2 | | Et |
| 1-141 | MeOCH₂ | HF₂CO | 0 | | Me |
| 1-142 | MeOCH₂ | HF₂CO | 1 | racemisch | Me |
| 1-143 | MeOCH₂ | HF₂CO | 1 | *(R)* | Me |
| 1-144 | MeOCH₂ | HF₂CO | 1 | (*S*) | Me |
| 1-145 | MeOCH₂ | HF₂CO | 2 | | Me |
| 1-146 | MeOCH₂ | HF₂CO | 0 | | Et |
| 1-147 | MeOCH₂ | HF₂CO | 1 | racemisch | Et |
| 1-148 | MeOCH₂ | HF₂CO | 1 | *(R)* | Et |
| 1-149 | MeOCH₂ | HF₂CO | 1 | (*S*) | Et |
| 1-150 | MeOCH₂ | HF₂CO | 2 | | Et |
| 1-151 | MeOCH₂ | F₃CO | 0 | | Me |
| 1-152 | MeOCH₂ | F₃CO | 1 | racemisch | Me |
| 1-153 | MeOCH₂ | F₃CO | 1 | *(R)* | Me |
| 1-154 | MeOCH₂ | F₃CO | 1 | (*S*) | Me |
| 1-155 | MeOCH₂ | F₃CO | 2 | | Me |
| 1-156 | MeOCH₂ | F₃CO | 0 | | Et |
| 1-157 | MeOCH₂ | F₃CO | 1 | racemisch | Et |
| 1-158 | MeOCH₂ | F₃CO | 1 | *(R)* | Et |
| 1-159 | MeOCH₂ | F₃CO | 1 | (*S*) | Et |
| 1-160 | MeOCH₂ | F₃CO | 2 | | Et |
| 1-161 | MeS | HF₂CO | 0 | | Me |
| 1-162 | MeS | HF₂CO | 1 | racemisch | Me |
| 1-163 | MeS | HF₂CO | 1 | *(R)* | Me |
| 1-164 | MeS | HF₂CO | 1 | (*S*) | Me |
| 1-165 | MeS | HF₂CO | 2 | | Me |
| 1-166 | MeS | HF₂CO | 0 | | Et |
| 1-167 | MeS | HF₂CO | 1 | racemisch | Et |
| 1-168 | MeS | HF₂CO | 1 | *(R)* | Et |
| 1-169 | MeS | HF₂CO | 1 | (*S*) | Et |
| 1-170 | MeS | HF₂CO | 2 | | Et |
| 1-171 | MeS | F₃CO | 0 | | Me |
| 1-172 | MeS | F₃CO | 1 | racemisch | Me |
| 1-173 | MeS | F₃CO | 1 | *(R)* | Me |
| 1-174 | MeS | F₃CO | 1 | (S) | Me |
| 1-175 | MeS | F₃CO | 2 | | Me |
| 1-176 | MeS | F₃CO | 0 | | Et |
| 1-177 | MeS | F₃CO | 1 | racemisch | Et |
| 1-178 | MeS | F₃CO | 1 | (*R*) | Et |
| 1-179 | MeS | F₃CO | 1 | (*S*) | Et |
| 1-180 | MeS | F₃CO | 2 | | Et |

### NMR-Daten ausgewählter Beispiele

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾;......; δₙ (Intensität.)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| 1-31: ¹H-NMR(400.6 MHz, CDC13): |
| δ= 8.1731 (2.7); 7.6629 (1.7); 7.6414 (2.0); 7.3533 (1.0); 7.3498 (1.1); 7.3319 (0.9); 7.3283 (0.9); 7.2619 (14.1); 2.4906 (0.6); 2.4651 (16.0); 0.0080 (0.6); -0.0002 (18.8); -0.0085 (0.5) |
| 1-21: ¹H-NMR(400.6 MHz, CDC13): |
| δ= 8.2180 (0.5); 7.7136 (0.9); 7.6922 (0.9); 7.2606 (42.9); 7.2510 (2.0); 7.2293 (1.2); 6.8380 (1.1); 6.6559 (2.3); 6.4739 (1.2); 2.4937 (1.9); 2.4744 (16.0); 1.5691 (1.0); 0.0079 (1.9); -0.0002 (60.1); -0.0085 (3.0); -0.0284 (0.5); -0.0410 (0.7) |
| 1-32: ¹H-NMR(400.6 MHz, CDC13): |
| δ= 7.5186 (1.1); 7.4207 (0.7); 7.3963 (0.7); 7.2602 (212.2); 6.9966 (1.1); 3.1170 (1.4); 3.1079 (16.0); 1.5462 (4.8); 1.2551 (1.3); 0.1458 (1.0); 0.0080 (8.1); -0.0002 (311.2); -0.0085 (10.1); -0.1495 (0.9) |
| 1-35: ¹H-NMR(400.6 MHz, CDC13): |
| δ= 7.8896 (0.6); 7.8679 (0.7); 7.5011 (0.5); 7.2605 (40.1); 3.3588 (16.0); 0.0079 (1.5); -0.0002 (60.1); -0.0060 (0.6); -0.0085 (1.8) |
| 1-22: ¹H-NMR(400.0 MHz, CD3CN): |
| δ= 8.4638 (2.4); 7.7968 (1.5); 7.7754 (1.7); 7.4144 (1.4); 7.3930 (1.3); 7.0952 (1.3); 6.9171 (1.1); 6.9050 (1.2); 6.7270 (1.3); 3.0905 (16.0); 2.1649 (4.1); 2.1355 (1.1); 2.1292 (0.8); 1.9923 (2.2); 1.9861 (1.6); 1.9804 (20.1); 1.9742 (40.6); 1.9680 (57.6); 1.9619 (41.1); 1.9557 (20.8) |
| 1-25: ¹H-NMR(400.0 MHz, CD3CN): |
| δ= 8.4656 (1.7); 7.8657 (1.2); 7.8442 (1.4); 7.4762 (0.7); 7.4738 (1.3); 7.4714 (0.8); 7.4548 (0.7); 7.4524 (1.2); 7.4500 (0.7); 7.0819 (1.3); 6.8997 (2.7); 6.7174 (1.3); 3.3750 (16.0); 3.1600 (0.8); 1.9922 (1.2); 1.9862 (0.7); 1.9803 (10.5); 1.9742 (20.5); 1.9679 (30.1); 1.9618 (20.0); 1.9556 (10.3); 1.1635 (2.4) |

### B. Formulierungsbeispiele

a) Ein Stäubemittel wird erhalten, indem man 10 Gew. Teile einer Verbindung der Formel (I) und/oder deren Salze und 90 Gew. Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew. Teile kaolinhaltigen Quarz als Inertstoff, 10 Gewichtsteile ligninsulfonsaures Kalium und 1 Gew. Teil oleoylmethyltaurinsaures Natrium als Netz und Dispergiermittel mischt und in einer Stiftmühle mahlt.
c) Ein in Wasser leicht dispergierbares Dispersionskonzentrat wird erhalten, indem man 20 Gew. Teile einer Verbindung der Formel (I) und/oder deren Salze mit 6 Gew. Teilen Alkylphenolpolyglykolether (^{®}Triton X 207), 3 Gew. Teilen Isotridecanolpolyglykolether (8 EO) und 71 Gew. Teilen paraffinischem Mineralöl (Siedebereich z.B. ca. 255°C bis über 277° C) mischt und in einer Reibkugelmühle auf eine Feinheit von unter 5 Mikron vermahlt.
d) Ein emulgierbares Konzentrat wird erhalten aus 15 Gew. Teilen einer Verbindung der Formel (I) und/oder deren Salze, 75 Gew. Teilen Cyclohexanon als Lösungsmittel und 10 Gew. Teilen oxethyliertes Nonylphenol als Emulgator.
e) Ein in Wasser dispergierbares Granulat wird erhalten indem man
   75 Gew. Teile einer Verbindung der Formel (I) und/oder deren Salze,
   10 Gew. Teile ligninsulfonsaures Calcium,
   5 Gew. Teile Natriumlaurylsulfat,
   3 Gew. Teile Polyvinylalkohol und
   7 Gew. Teile Kaolin
   mischt, auf einer Stiftmühle mahlt und das Pulver in einem Wirbelbett durch Aufsprühen von
   Wasser als Granulierflüssigkeit granuliert.
f) Ein in Wasser dispergierbares Granulat wird auch erhalten, indem man
   25 Gew. Teile einer Verbindung der Formel (I) und/oder deren Salze,
   5 Gew. Teile 2,2' Dinaphthylmethan 6,6' disulfonsaures Natrium,
   2 Gew. Teile oleoylmethyltaurinsaures Natrium,
   1 Gew. Teil Polyvinylalkohol,
   17 Gew. Teile Calciumcarbonat und
   50 Gew. Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Daten

Die nachfolgend genutzten Abkürzungen bedeuten:
Unerwünschte Pflanzen:

| | | | |
|---|---|---|---|
| ALOMY: | Alopecurus myosuroides | SETVI: | Setaria viridis |
| AMARE: | Amaranthus retroflexus | AVEFA: | Avena fatua |
| LOLRI: | Lolium rigidum | ECHCG: | Echinochloa crus-galli |
| VERPE: | Veronica persica | VIOTR: | Viola tricolor |
| POLCO: | Polygonum convolvulus | ABUTH: | Abutylon threophrasti |
| PHBPU: | Pharbitis purpurea | MATIN: | Matricaria inodora |
| DIGSA | Digitaria sanguinalis | KCHSC: | Kochia scoparia |

### 1. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

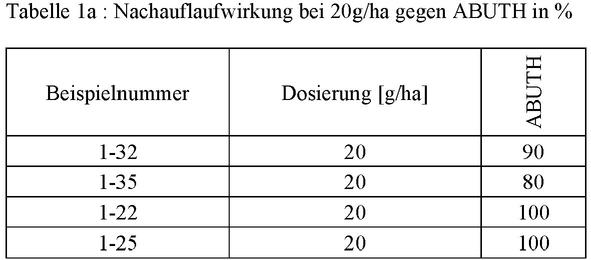

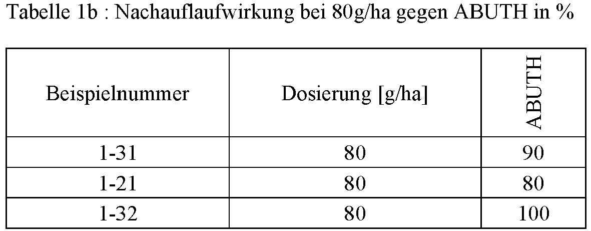

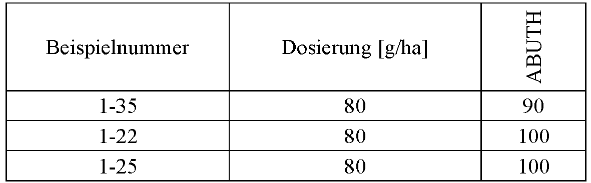

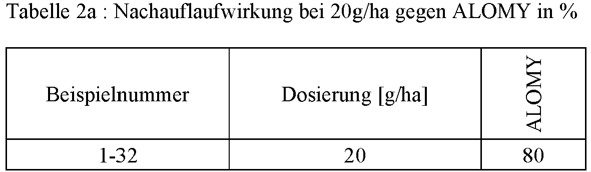

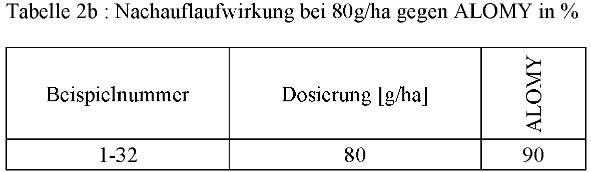

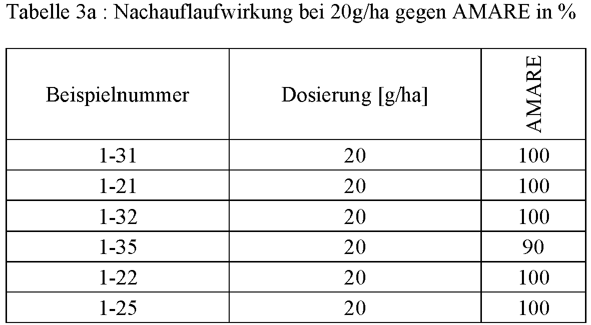

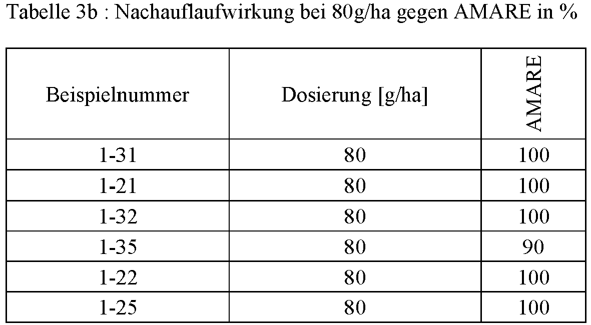

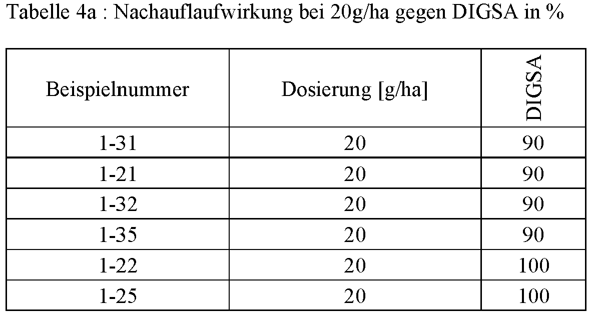

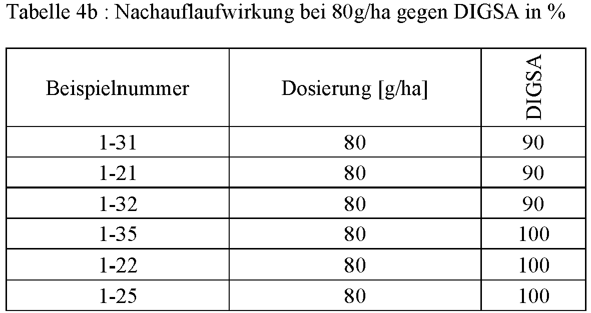

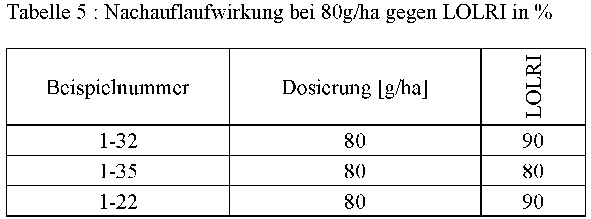

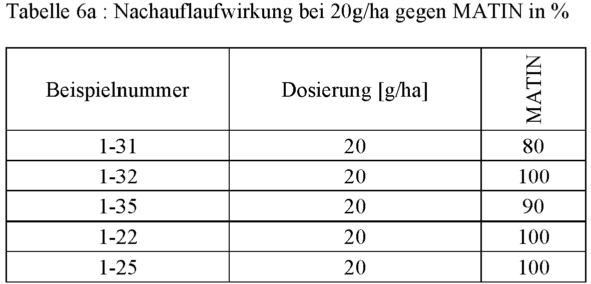

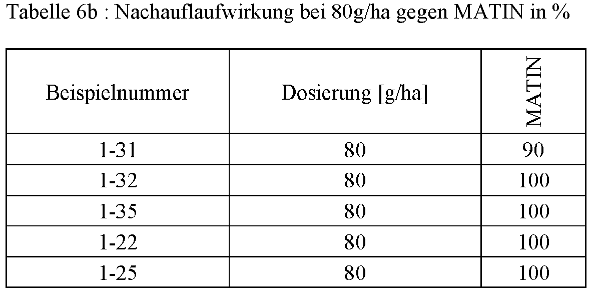

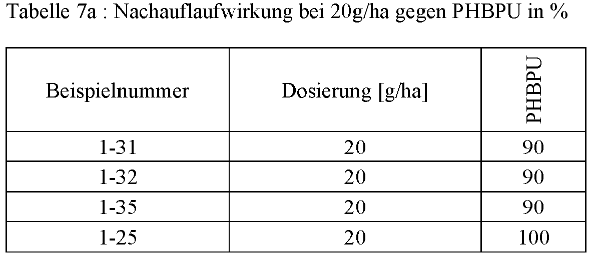

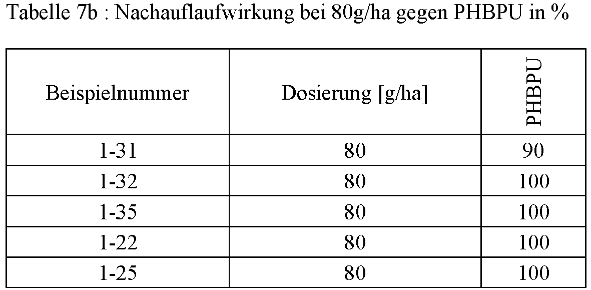

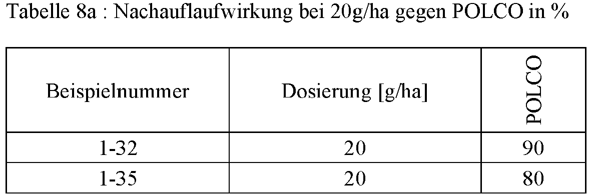

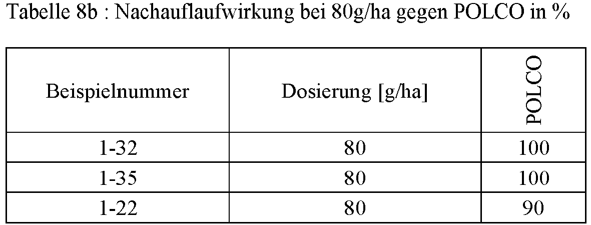

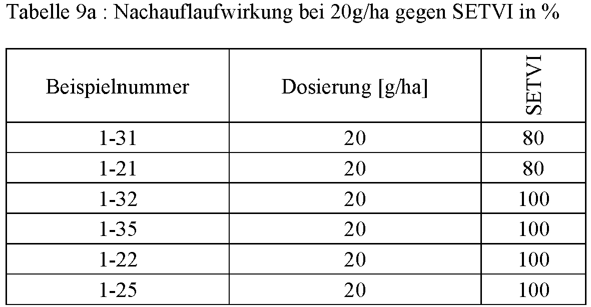

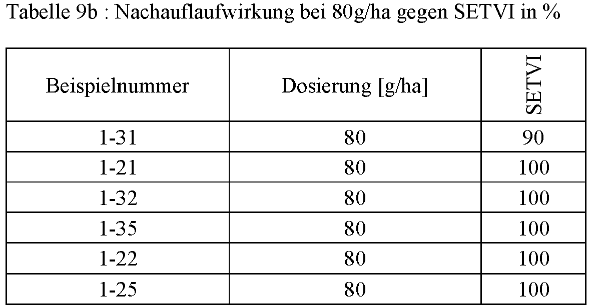

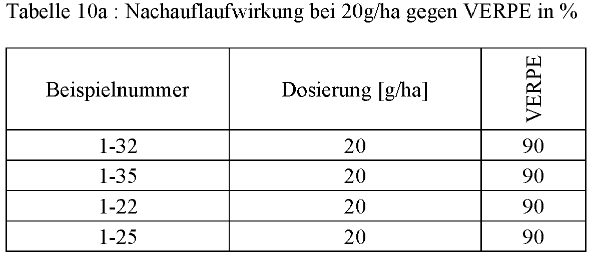

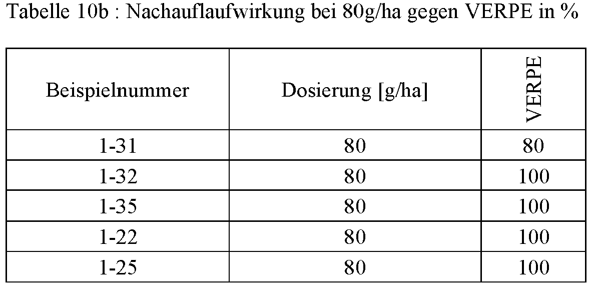

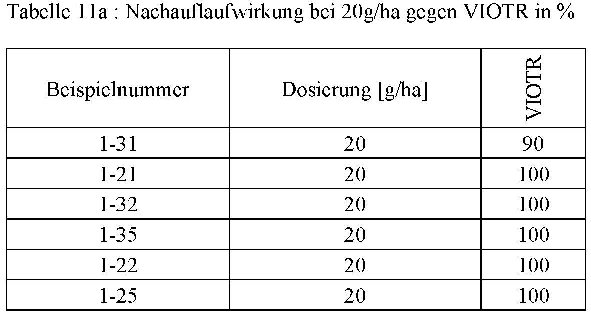

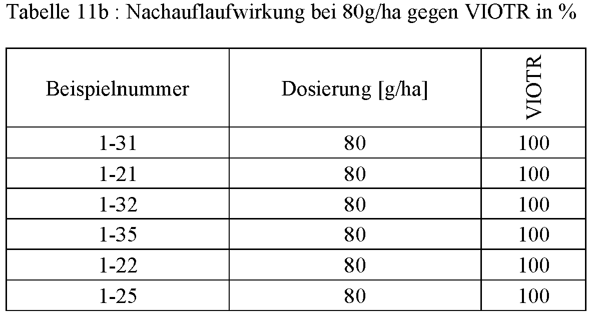

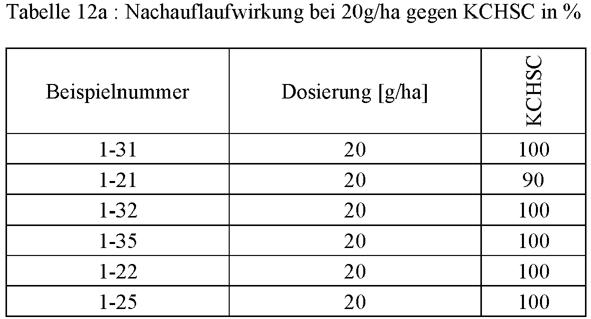

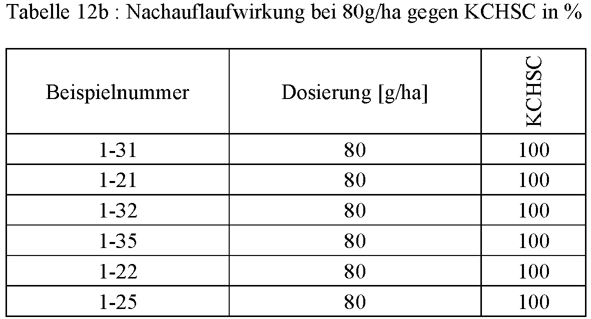

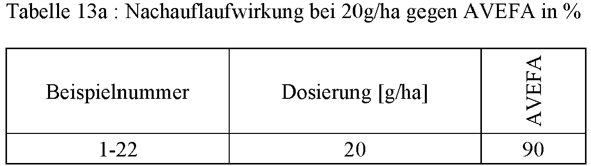

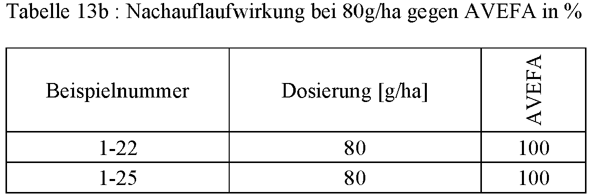

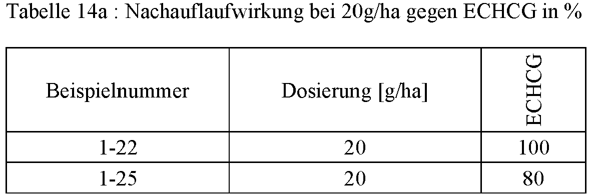

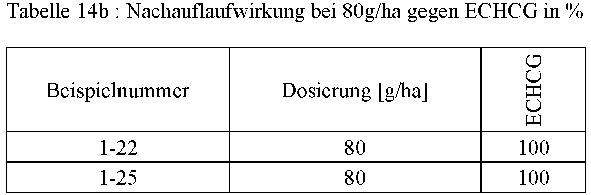

Wie die Ergebnisse aus Tabellen 1a/b, 2a/b, 3a/b, 4a/b, 5, 6a/b, 7a/b, 8a/b, 9a/b, 10a/b, 11a/b, 12a/b, 13a/b und 14a/b zeigen, weisen die erfindungsgemäßen Verbindungen eine gute herbizide Nachauflaufwirksamkeit gegen ein breites Spektrum von Ungräsern und Unkräutern auf. Beispielsweise zeigen die aufgeführten Beispiele bei einer Aufwandmenge von 80/20 g/ha eine 80 - 100%-ige Wirkung unter anderem gegen *Alopecurus myosuroides, Digitaria sanguinalis, Setaria viridis, Veronica persica* und *Viola tricolor.* Die erfindungsgemäßen Verbindungen eignen sich deshalb im Nachauflaufverfahren zur Bekämpfung von unerwünschtem Pflanzenwuchs.

### 2. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 l/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen).

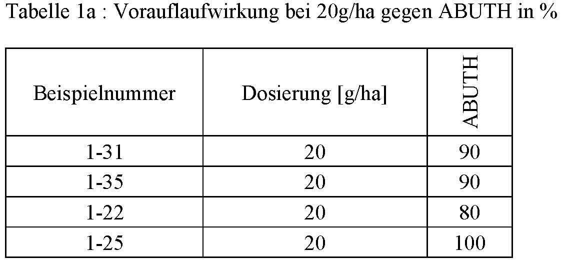

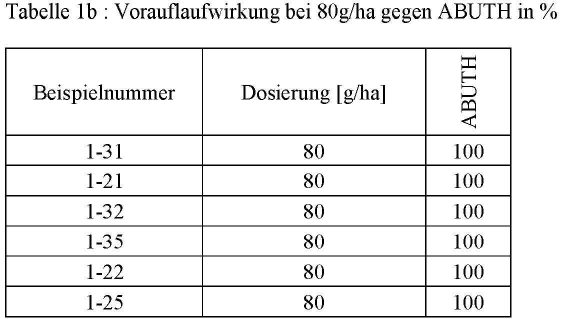

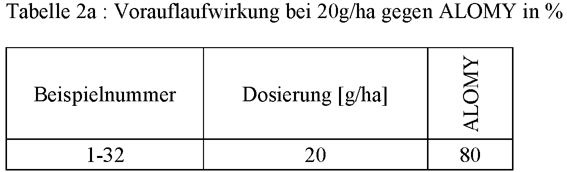

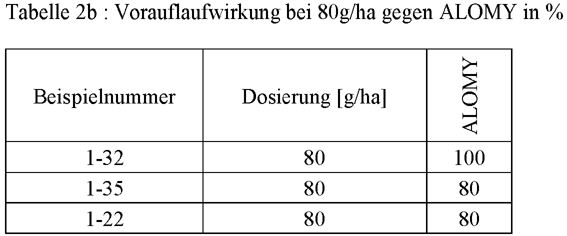

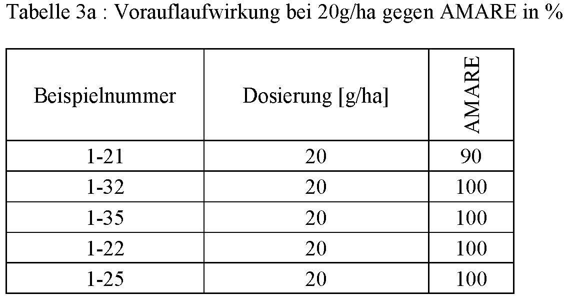

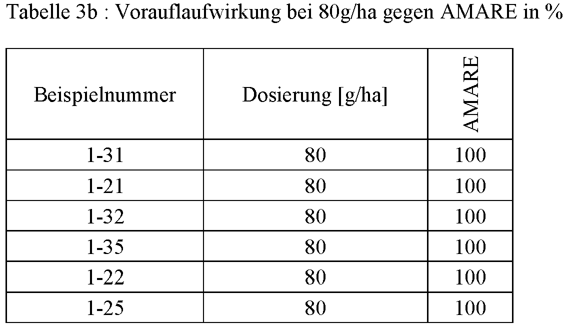

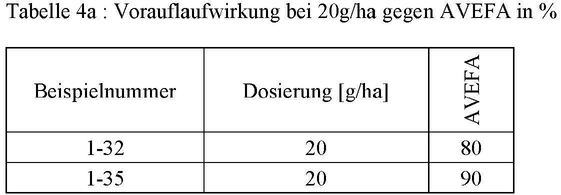

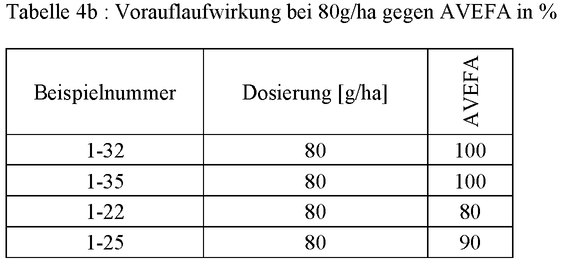

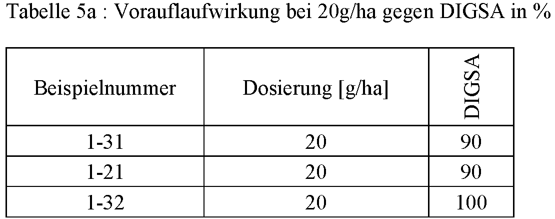

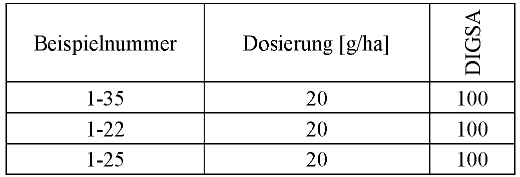

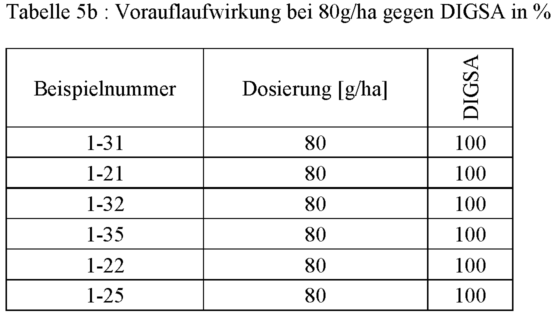

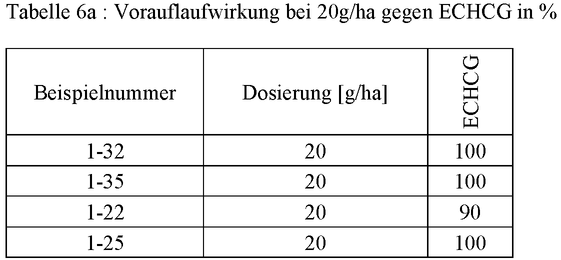

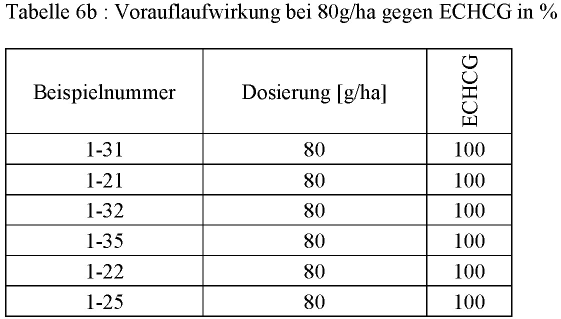

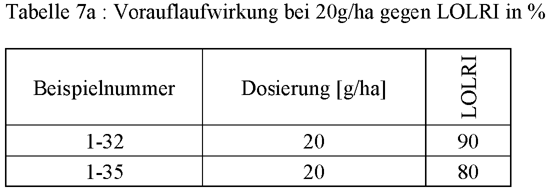

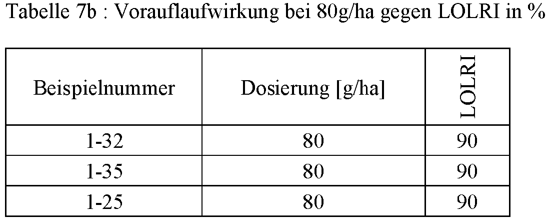

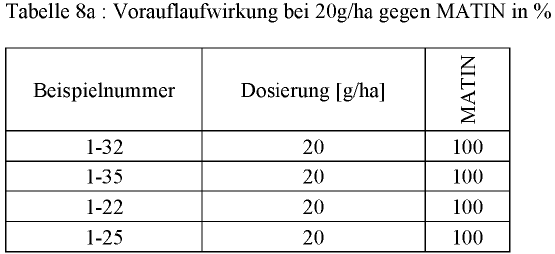

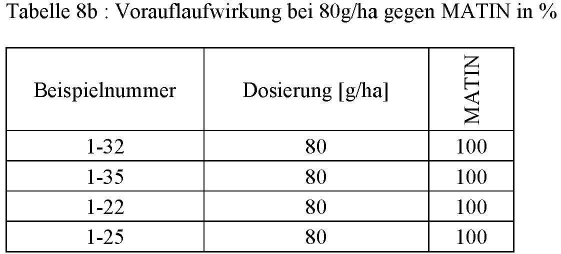

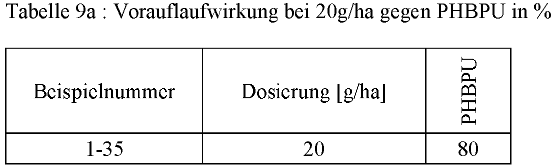

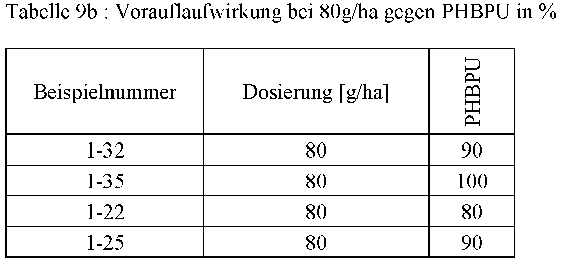

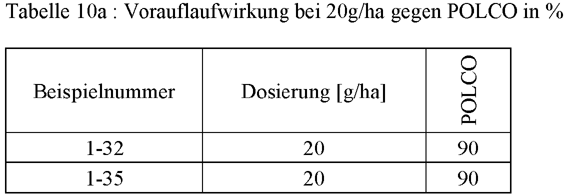

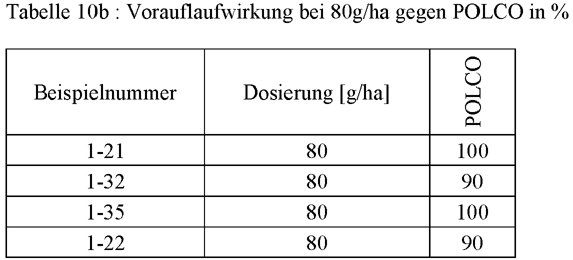

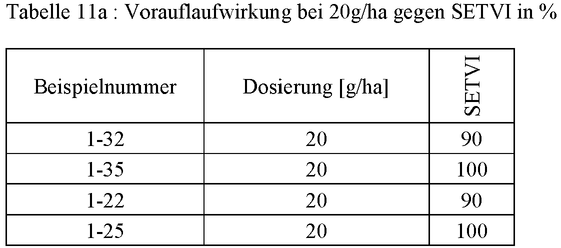

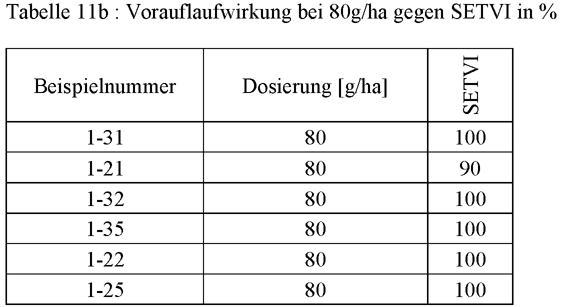

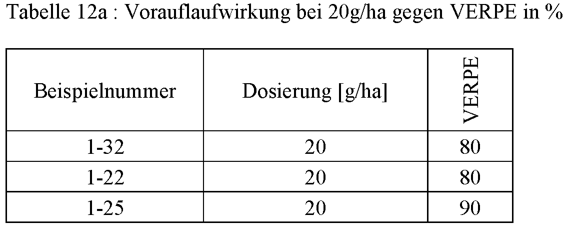

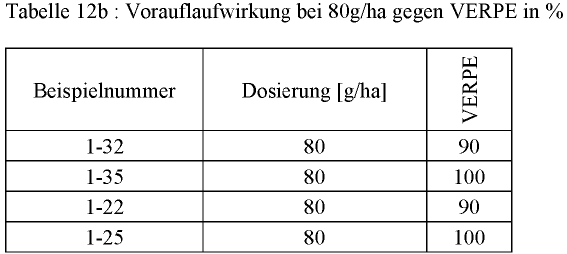

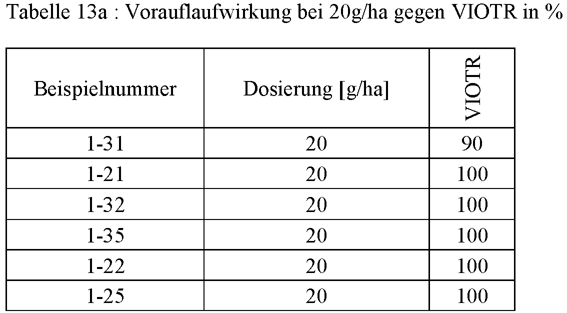

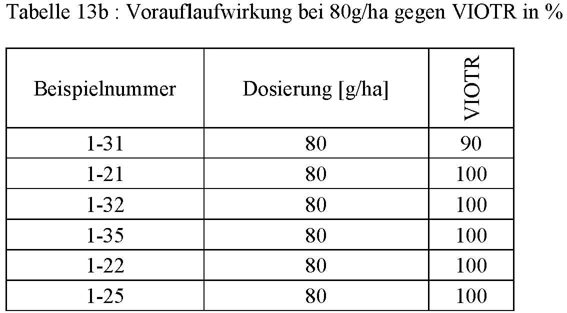

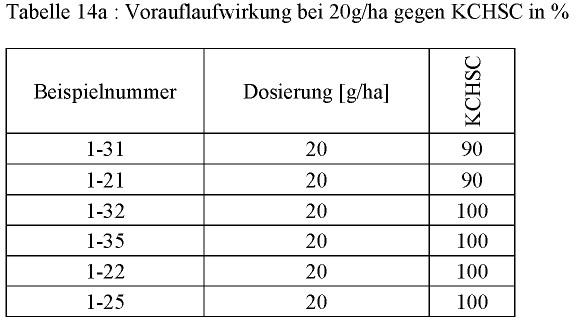

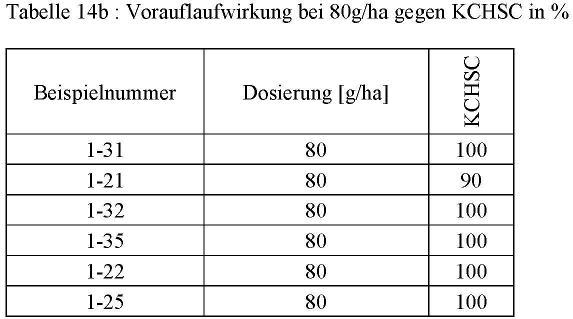

Wie die Ergebnisse aus Tabellen 1a/b, 2a/b, 3a/b, 4a/b, 5a/b, 6a/b, 7a/b, 8a/b, 9a/b, 10a/b, 11a/b, 12a/b, 13a/b und 14a/b zeigen, weisen die erfindungsgemäßen Verbindungen eine gute herbizide Vorauflaufwirksamkeit gegen ein breites Spektrum von Ungräsern und Unkräutern auf. Beispielsweise zeigen die Verbindungen bei einer Aufwandmenge von 80/20 g/ha jeweils eine 80 - 100%-ige Wirkung unter anderem gegen *Alopecurus myosuroides, Avena fatua, Digitaria sanguinalis, Echinochloa crus-galli, Lolium rigidum, Setaria viridis, Amaranthus retroflexus, Viola tricolor* und *Veronica persica.* Die erfindungsgemäßen Verbindungen eignen sich deshalb im Vorauflaufverfahren zur Bekämpfung von unerwünschtem Pflanzenwuchs.

## Patentansprüche

1. Benzoesäureamide der Formel (I) oder deren Salze 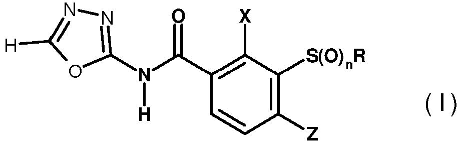 worin die Symbole und Indizes folgende Bedeutungen haben:
X bedeutet Cl, Br, Me, Et, MeO, EtO, MeOCH₂ oder MeS,
Z bedeutet HF₂CO oder F₃CO,
R bedeutet Me oder Et,
n bedeutet 0, 1 oder 2.

2. Herbizides Mittel oder pflanzenwachstumsregulierendes Mittel, **dadurch gekennzeichnet, dass** es eine oder mehrere Benzoesäureamide der allgemeinen Formel (I) oder deren Salze nach Anspruch 1 enthält.

3. Herbizide Mittel nach Anspruch 2, die ferner ein Formulierungshilfsmittel enthalten.

4. Herbizide Mittel nach Anspruch 2 oder 3 enthaltend mindestens einen weiteren Wirkstoff aus der Gruppe der Insektizide, Akarizide, Herbizide, Fungizide, Safenern und/oder Wachstumsregulatoren.

5. Herbizide Mittel nach Anspruch 2 oder 3 enthaltend einen Safener.

6. Herbizide Mittel nach Anspruch 5, worin der Safener ausgewählt ist aus der Gruppe bestehend aus Mefenpyr-diethyl, Cyprosulfamid, Isoxadifen-ethyl, Cloquintocet-mexyl, Benoxacor und Dichlormid.

7. Verfahren zur Bekämpfung unerwünschter Pflanzen, **dadurch gekennzeichnet, dass** man eine wirksame Menge mindestens eines Benzoesäureamids der Formel (I) gemäß Anspruch 1 oder eines herbiziden Mittels nach einem der Ansprüche 2 bis 6 auf die Pflanzen oder auf den Ort des unerwünschten Pflanzenwachstums appliziert.

8. Verwendung von Benzoesäureamiden der Formel (I) gemäß Anspruch 1 oder von herbiziden Mitteln nach einem der Ansprüche 2 bis 6 zur Bekämpfung unerwünschter Pflanzen.

9. Verwendung nach Anspruch 8, **dadurch gekennzeichnet, dass** die Benzoesäureamide der Formel (I) zur Bekämpfung unerwünschter Pflanzen in Kulturen von Nutzpflanzen eingesetzt werden.

10. Verwendung nach Anspruch 9, **dadurch gekennzeichnet, dass** die Nutzpflanzen transgene Nutzpflanzen sind.

## Claims

1. Benzoic acid amides of formula (I) or salts thereof 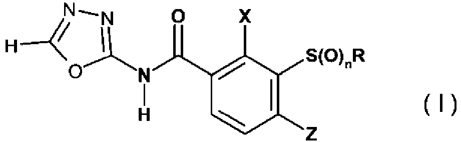 where the symbols and indices have the following meanings:
X represents Cl, Br, Me, Et, MeO, EtO, MeOCH₂ or MeS,
Z represents HF₂CO or F₃CO,
R represents Me or Et,
n represents 0, 1 or 2.

2. A herbicidal composition or plant growth regulating composition, **characterized in that** it contains one or more benzoic acid amides of the general formula (I) or salts thereof as defined in claim 1.

3. Herbicidal compositions of claim 2, further containing a formulation aid.

4. Herbicidal compositions of claim 2 or 3, containing at least one further active ingredient from the group comprising insecticides, acaricides, herbicides, fungicides, safeners and/or growth regulators.

5. Herbicidal compositions of claim 2 or 3, containing a safener.

6. Herbicidal compositions of claim 5, wherein the safener is selected from the group consisting of mefenpyr-diethyl, cyprosulfamide, isoxadifen-ethyl, cloquintocet-mexyl, benoxacor and dichlormid.

7. A method for controlling unwanted plants, **characterized in that** an effective amount of at least one benzoic acid amide of formula (I) according to claim 1 or a herbicidal composition of claim 2 to 6 is applied to the plants or to the site of the unwanted plant growth.

8. Use of benzoic acid amides of formula (I) as defined in claim 1, or of herbicidal compositions of claim 2 to 6, for controlling unwanted plants.

9. Use according to claim 8, **characterized in that** the benzoic acid amides of formula (I) are used for controlling unwanted plants in crops of useful plants.

10. Use according to claim 9, **characterized in that** the useful plants are transgenic useful plants.

## Revendications

1. Les amides de l'acide benzoïque de formule (I) ou leurs sels 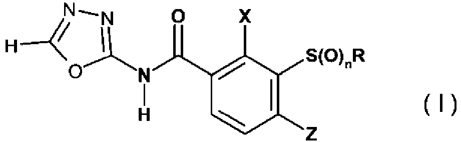 dans laquelle les symboles et les indices ont les significations suivantes :
X représente CI, Br, Me, Et, MeO, EtO, MeOCH₂ ou MeS,
Z représente HF₂CO ou F₃CO,
R représente Me ou Et,
n représente 0, 1 ou 2.

2. Produit herbicide ou régulateur de croissance des plantes, **caractérisé en ce qu'**il contient un ou plusieurs amides de l'acide benzoïque de formule générale (I) ou leurs sels, selon la revendication 1.

3. Produit herbicide selon la revendication 2, contenant en outre un adjuvant de formulation.

4. Produit herbicide selon la revendication 2 ou 3, contenant au moins une autre substance active choisie parmi le groupe comprenant les insecticides, les acaricides, les herbicides, les fongicides, les phytoprotecteurs et/ou les régulateurs de croissance.

5. Produit herbicide selon la revendication 2 ou 3, contenant un phytoprotecteur.

6. Produit herbicide selon la revendication 5, dans lequel le phytoprotecteur est sélectionné parmi le groupe constitué du méfenpyr-diéthyle, du cyprosulfamide, de l'isoxadifène-éthyle, du cloquintocet-mexyle, du bénocacor et du dichlormide.

7. Procédé de lutte contre les plantes indésirables, **caractérisé en ce qu'**il comprend l'application d'une quantité efficace d'au moins un amide d'acide benzoïque de formule (I) selon la revendication 1 ou d'un produit herbicide selon l'une des revendications 2 à 6 sur les plantes ou sur le lieu de croissance des plantes indésirables.

8. Utilisation d'amides d'acide benzoïque de formule (I) selon la revendication 1 ou de produits herbicides selon l'une des revendications 2 à 6 pour lutter contre les plantes indésirables.

9. Utilisation selon la revendication 8, **caractérisée en ce que** les amides de l'acide benzoïque de formule (I) sont utilisés pour lutter contre les plantes indésirables dans les cultures de plantes utiles.

10. Utilisation selon la revendication 9, **caractérisée en ce que** les plantes utiles sont des plantes utiles transgéniques.
